# EUROPEAN PATENT SPECIFICATION

(11) **EP 2 057 137 B1**
(45) Date of publication and mention of the grant of the patent: **23.10.2013**
(21) Application number: 07805638.9
(22) Date of filing: 02.08.2007
(51) Int. Cl.: C07D 311/30, A61K 31/352, A61P 3/10

(54) **S-(+)-7-[3-N-SUBSTITUTED AMINO-2-HYDROXYPROPOXY]FLAVONES**
S-(+)-7-[3-N-SUBSTITUIERTE AMINO-2-HYDROXYPROPOXY]FLAVONE
S-(+)-7-[3-N-(AMINO SUBSTITUÉ)-2-HYDROXYPROPOXY]FLAVONES

(30) Priority: 09.08.2006 IN DE18032006
(43) Date of publication of application: 13.05.2009
(73) Proprietor: Council of Scientific and Industrial Research, New Delhi - 110 001 (IN)
(72) Inventor: PRATAP, Ram, Uttar Pradesh (IN); SINGH, Himanshu, Uttar Pradesh (IN); VERMA, Alok, Kumar, Uttar Pradesh (IN); SINGH, Amar, Bahadur, Uttar Pradesh (IN); TIWARI, Priti, Uttar Pradesh (IN); SRIVASTAVA, Mukesh, Uttar Pradesh (IN); SRIVASTAVA, Arvind, Kumar, Uttar Pradesh (IN); DWIVEDI, Anil, Kumar, Uttar Pradesh (IN); SINGH, Satyawan, Uttar Pradesh (IN); SRIVASTAVA, Pratima, Uttar Pradesh (IN); SINGH, Shio, Kumar, Uttar Pradesh (IN); NATH, Chandishwar, Uttar Pradesh (IN); RAGHUBIR, Ram, Uttar Pradesh (IN)
(74) Representative: Bond, Christopher William
(86) International application number: PCT/IN2007/000326
(87) International publication number: WO 2008/018089

(56) References cited:
- EP-A- 0 064 165
- EP-A- 0 288 077
- WO-A-2006/040621
- E. S. C. WU ET AL: "Flavones. 3. Synthesis, Biological Activities, and Conformational analysis of Isoflavone Derivatives and Related Compounds" J. MED. CHEM., vol. 35, 1992, pages 3519-3525, XP002460985
- HE X ET AL: "SYNTHESIS OF FLAVONE AND FLAVANONE-7-O-ISOPROPANOLAMINE DERIVATIVES AND THEIR ANTIARRHYTHMIC ACTIVITY" ZHONGGUO YAOWU HUAXUE ZAZHI - CHINESE JOURNAL OF MEDICINAL CHEMISTRY, GAI-KAI BIANJIBU, SHENYANG, CN, vol. 1, no. 1, 1990, pages 11-20, XP009054460 ISSN: 1005-0108
- WU E S C ET AL: "FLAVONES 2. SYNTHESIS AND STRUCTURE-ACTIVITY RELATIONSHIP OF FLAVODILOL AND ITS ANALOGUES A NOVEL CLASS OF ANTIHYPERTENSIVE AGENTS WITH CATECHOLAMINE DEPLETING PROPERTIES" JOURNAL OF MEDICINAL CHEMISTRY, AMERICAN CHEMICAL SOCIETY. WASHINGTON, US, vol. 32, no. 1, 1989, pages 183-192, XP002346714 ISSN: 0022-2623
- WANG DING ET AL: "SYNTHESIS OF SOME (2-HYDROXY-3-AMINOPROPOXY) FLAVONES" YAO HSUEH HSUEH PAO - ACTA PHARMACEUTICA SINICA, BEIJING, CN, vol. 15, no. 4, 1980, pages 253-256, XP009054462 ISSN: 0513-4870

## Description

### Field of Invention

The present invention relates to S-(+)-7-[3-N- substituted amino-2-hydroxypropoxy] flavones and salts thereof. The present invention relates to synthesis of suitably substituted flavone derivatives exhibiting pronounced antidiabetic and antidyslipidemic activities. More particularly the invention relates to synthesis of S-(+)-7-[3 N-substitutedamino-2-hydroxypropoxy] flavones of formula I and pharmaceutical composition containing these derivatives, as described in the following description.

### Background of the Invention and Prior art

Type II insulin resistant diabetes mellitus accounts for 90-95% of all diabetes. Changed sedentary life style has contributed towards affliction of the disease to adult population also. The main force driving this increasing incidence is a staggering increase in obesity, the single most important contributor to the pathogenesis of diabetes mellitus. Prolonged disease condition leads to chronic macrovascular complications such as retinopathy and nephropathy. The disease is collectively referred, as metabolic syndrome encompasses type II diabetes and common constellation of closely linked clinical features. Characteristic factors include insulin resistance *per se,* obesity, hypertension and a common form of dyslipidemia and low high-density lipoprotein cholesterol. Metabolic syndrome is associated with marked increased incidence of coronary, cerebral and peripheral artery disease [Executive summary of the third report of the National Cholesterol Program Expert Panel on detection, evaluation and treatment of high blood cholesterol in adults (2001), J. Am. Med. Assoc. 285, 2486-2496].

The role of peripheral and hepatic insulin resistance in the pathogenesis of diabetes mellitus is undisputed. Insulin resistance can be due to multiple defects in signal transduction such as impaired activation of insulin receptor-tyrosine kinase and reduced activation of insulin-stimulated phosphatidyl inositol-3-hydroxy kinase. The resistance of insulin due to diet-induced obesity [Elchebly, M. et al. (1999), Science, 283, 1544.] has given the critical role of obesity in the development of insulin resistance and other features of the metabolic syndrome. Successful approaches attenuating appetite and/or enhancing energy expenditure will prove to be of great benefit in preventing and treating type II diabetes. Abnormalities of fatty acid metabolism are increasingly recognized as key components of the pathogenesis of the metabolic syndrome and type II diabetes. A critical player in potentiating the promoting effect of hyperinsulinaemia on hepatic lipid accumulation is the anabolic transcription factor SREBP-1, which upregulates genes such as that for fatty acid synthase [Shimomura, I. et al. (2000), Mol. Cell, 6, 77-86.]. These observations support a unified "lipotoxicity" hypothesis, which states that metabolic syndrome and type II diabetes can be caused by the accumulation of triglycerides and long chain fatty-acyl-CoA in liver and muscle. The third causal factor of metabolic syndrome is oxidative stress. Excess levels of oxygen in the living body can also pose a serious health threat; the so-called oxygen toxicity is brought about by oxygen species such as hydrogen peroxide and oxy radicals and damage living tissue. The active oxygen species are associated with diabetes mellitus and are destructive towards various tissues as occurring in diabetes mellitus. There have been many reports discussing relationships between peroxidation and diseases such as diabetes mellitus, atherosclerosis and myocardial ischemia in terms of radical oxidation. Glucose is oxidized under oxidative stress to highly reactive species, which ultimately reacts with proteins. Glucose, like other α-hydroxy aldehydes, can enolize and thereby reduce molecular oxygen under physiological conditions, catalyzed by transition metals, yielding α-keto aldehydes and oxidizing intermediates. These secondary compounds are more reactive than monosaccharides and can react with proteins to form cross-linked Maillard products (Simon P. Wolff et al. 1991; Free Radical Biology and Medicine, 10, 339-352.).

Oxidative stress also modifies lipids. Like glucose, LDL also undergoes oxidative modification to form modified LDL (oxidized LDL). The actual oxidation process is believed to begin with lipid peroxidation, followed by fragmentation to give short chain aldehydes. These aldehydes in turn react with the lysine residues of apo-B, creating a new epitope, which is recognized by the scavenger receptor of macrophages. During this same process, lecithin is converted to lysolecithin, which is a selective chemotactic agent for monocytes. The monocytes enter the subendothelium and undergo a phenotypic change to a macrophage, which avidly take up the oxidized LDL via the scavenger receptor. The uptake of oxidized LDL continues until the macrophage is so engorged with cholesteryl esters that it transforms into a foam cell. Groups of these foam cells constitute a fatty streak, the earliest hallmark of atherosclerosis. By inhibiting the oxidation of LDL, it is hoped that the modification of apo-B and the production of chemotactic lysolecithin can be prevented and in-turn the atherosclerosis.

At present, therapy for type II diabetes relies mainly on several approaches intended to reduce the hyperglycemia itself: sulphonylureas which increase insulin secretion from pancreatic beta cells; Metformin which acts to reduce hepatic glucose production, peroxisome proliferator activated receptor-y agonists which enhance insulin action and α-glucosidase inhibitors interfere with gut glucose absorption. These therapies have limited efficacy, limited tolerability and mechanism-based toxicity. Of particular concern is the tendency for most treatments to enhance weight gain. A problem particular to the sulphonylureas is that many patients who respond initially become refractory to treatment overtime.

The increasing prevalence of obesity and its associated co-morbidities including type II diabetes and related cardiovascular disorders has stimulated efforts to develop effective new approaches in the treatment of this condition. While most therapeutic approaches involve altering the balance of metabolic energy by reducing energy intake, an alternative approach for the management of obesity is to affect an increase in the rate of energy expenditure. In 1984, compounds of the phenethanolamine class as shown below having thermogenic properties in rodents were first disclosed. Despite their structural similarity to known β₁ and β₂ adrenoceptor ligands, pharmacological studies indicated that these compounds stimulated a third or 'atypical' β-adrenergic receptor (β-AR) that is now described as β₃-AR. β₃ agonist also increased insulin sensitivity and glucose utilization. Later studies suggested that Tyr 64 Arg β₃-AR mutation in the human population plays a role in the development of diabetes mellitus and/or obesity in some individuals possessing this genetic variant [Turner, N. C.; (1996), DDT, 1, 109-116]. Among phenethanolamine compounds as thermogenic agent acting on β₃-adrenergic receptor, CL-316,243 is exhibiting promising activity in humans.

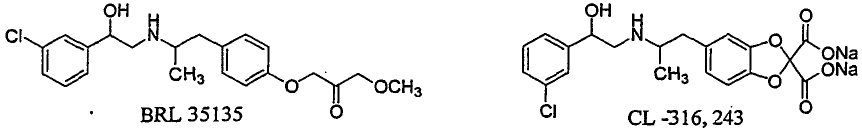

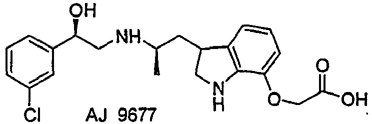

The paradigm of current drug design has shifted from single target to multi target drug called network models which suggest that partial inhibition of a surprisingly small number of targets can be more efficient than the complete inhibition of a single target. Troglitazone is such a drug which had pharmacophores to focus two targets however it failed because of its toxic metabolites. In order to design drug for metabolic syndrome X we targeted oxidative stress and stimulation of thermogenesis. We tried to amalgamate the pharmacophore for above targets in a single molecule. Flavonoids were selected as basic skeleton to attach phamacophore for thermogenesis. Flavonoids are among the most ubiquitous groups of polyphenolic compounds in foods of plant origin. As integral constituents of the diet, they may exert a wide range of beneficial effects on human health. Flavonoids produce such biological effects through their free radical scavenging antioxidant activities and metal ion chelating abilities. (Cotelle, N. et al, Free Rad. Biol. Med. 1992, 13, 211). These properties led us to utilize flavones for the synthesis of hybrid molecules as antidiabetic and antidyslipidemic agents by substitution with thermogenic as well as insulin sensitizing pharmacophores. In our earlier application we have made claim for 7-N substituted 2-hydoxy propoxy flavone as potent antilipidemic and antidyslipidemic agent. Here we claim antidiabetic and antidyslipidemic activity of their optical enantiomers.

WO 2006/040621 A, Council Scient Ind Res, Pratap Ram *et al.,* 20 April 2006 discloses substituted flavone derivatives which exhibit anti hyperglycaemic and antidyslipidemic activity.

E. S. C. Wu et al., "Flavones. 3. Synthesis, Biological Activities, and Conformational analysis of Isoflavone Derivatives and Related Compounds", J. Med. Chem., vol. 35, 1992, pages 3519-3525, discloses a series of 2-alkylisoflavone derivatives and studies their antihypertensive activity.

He X et al., "Synthesis Of Flavone And Flavanone-7-O-Isopropanolamine Derivatives And Their Antiarrhythmic Activity", Zhongguo Yaowu Huaxue Zazhi - Chinese Journal Of Medical Chemistry, Gai-Kai Bianjibu, Shenyang, CN, vol. 1, no. 1, 1990, pages 11-20, discloses flavone derivatives and studies their antiarrythmic activity.

EP-A-0 288 077 (Pennwalt Corp), 26 October 1988, discloses 7-[3-[(3,4-dihydroxyphenethyl)amino]-2-hydroxypropoxy]flavone hydrobromide in the treatment of rat anaphylaxis.

Wu E S C et al., "Flavones 2. Synthesis And Structure-Activity Relationship Of Flavodilol And Its Analogues A Novel Class Of Antihypertensive Agents With Catecholamine Depleting Properties", Journal Of Medicinal Chemistry, American Chemical Society, Washington, US, vol. 32, no. 1, 1989, pages 183-192, discloses a series of flavonoxypropanolamines and studies their antihypertensive activity.

EP-A-0 064 165 (Pennwalt Corp) 10 November 1982 discloses 7-(3-n-propylamino-2-hydroxypropoxy)-2,3-diphenylchromone and studies its effect as an antihypertensive agent.

Wang Ding et al., "Synthesis Of Some (2-Hydroxy-3-Aminopropoxy) Flavones", Yao Hsueh Hsueh Pao - Acta Pharmaceutica Sinica, Beijing, CN, vol. 15, no. 4, 1980, pages 253-256, discloses the preparation of (2-Hydroxy-3-Aminopropoxy) Flavones.

### Objects of the Present Invention

The main objective of the present invention is to provide S-(+)-7-[3-N-substituted amino-2-hydroxypropoxy] flavones and salts thereof.

Another object of the present invention is to provide process for preparation of compound of formula **I.**

Yet another object of the present invention is to provide a pharmaceutical composition containing these flavone derivatives and a pharmaceutically acceptable carrier or diluent thereof.

Yet another object of the present invention is to provide a method for treating type II diabetes and associated hyperlipidemic conditions in a mammal by administering a pharmaceutically acceptable amount of compound of formula I optionally along with pharmaceutically acceptable excipents.

### Summary of the Invention

The present invention is as defined in the claims. The present invention will now be defined with reference to the following clauses:
1. An optically active compound selected from the group consisting of:
   (a) S-(+)-7-(3-tert-Butylamino-2-hydroxy-propoxy)-3',5'-dibenzyloxy-flavone (7);
   (b) S-(+)-7-(3-iso-Propylamino-2-hydroxy-propoxy)-3', 5'-dibenzyloxy- flavone (10); or
   (c) S-(+)-7-(3-iso-Propylamino-2-hydroxy-propoxy)-3', 5'-dihydroxy - flavone (11).
2. A compound according to clause 1, wherein the compound is; 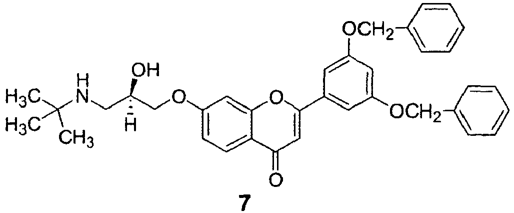
3. A compound according to clause 1, wherein the compound is; 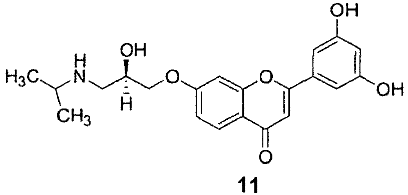
4. A compound according to clause 1, wherein the salt of the compound is selected from group consisting of hydrochloride, succinates, fumarates and tartrates.
5. A compound according to clause 1, wherein the compound exhibits antidiabetic and lipid lowering activity at a dose ranging between 10-250 mg /Kg-body weight.
6. A compound according to any one of clauses 1 to 5 for use in a method of treating type II diabetes and dislipidemia in mammals, said method comprising the step of administering a pharmaceutically effective amount of the compound, optionally with the pharmaceutically acceptable exciepients.
The invention also provides a method for controlling 'type II' diabetes and associated hyperlipidemic conditions in a mammal by administering these compounds and compositions containing these derivatives.

### Detailed Description of the Invention

Accordingly, the present disclosure provides an optically active compound of general formula I and salts thereof: 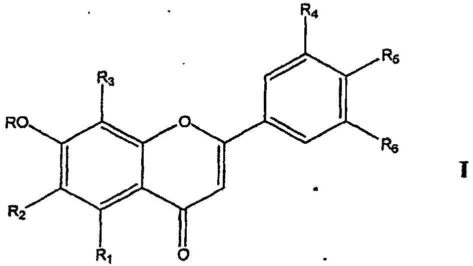 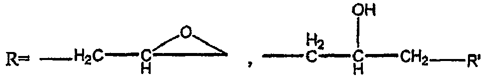 wherein R' is selected from a group consisting of t-butyl amine, n-butylamine, isobutylamine, iso-propyl amine, 4-phenyl-piperazine-1-ylamine, 4-(2-methoxyphenyl)-piperazin-1-ylamine, and 3, 4-dimethoxy phenethyl amine;
wherein R₁, R₂ and R₃ are selected from the group consisting of hydrogen, methyl or iso pentenyl; R₄, R₅ and R₆ are selected from the group consisting of OH, O-alkyl, O-benzyl, O-substituted phenyl or combination thereof.

Another aspect of the present disclosure provides a pharmaceutical composition comprising the compound of formula I and pharmaceutically acceptable quantities of a conventional pharmaceutically acceptable carrier or diluent thereof.

In yet another aspect of the present disclosure, the compound of formula I is used along with pharmaceutically acceptable quantities of conventional lipid lowering agents and / or conventional sugar lowering agents.

Yet another aspect of the present disclosure provides a method for treating type II diabetes and associated hyperlipidemic conditions in mammals by administering a pharmaceutically effective amount of compound of formula I, optionally with other conventional diabetic and lipid lowering agents.

Yet another aspect of the present disclosure provides a method of treating macrovascular conditions such as retinopathy and nephropathy in mammals by administering a pharmaceutically effective amount of the compound of formula I, optionally with other conventional diabetic and lipid lowering agents.

In yet another aspect of the present disclosure, the range of pharmaceutically effective dose is 10-250 mg / Kg body weight of the compound.

Still another aspect of the present disclosure provides a compound of formula (4) of configuration R.

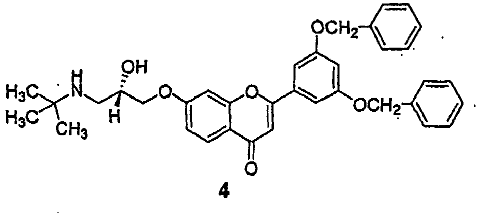

Still another aspect of the present disclosure provides a compound of formula (7) of configuration S.

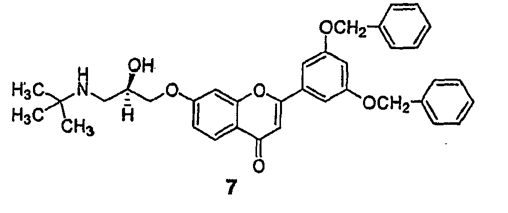

Yet another aspect of the present disclosure provides a compound of formula (9) of configuration R.

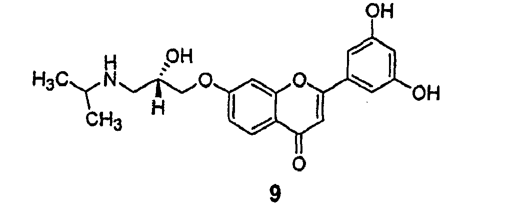

Yet another aspect of the present disclosure provides a compound of formula (11) of configuration S.

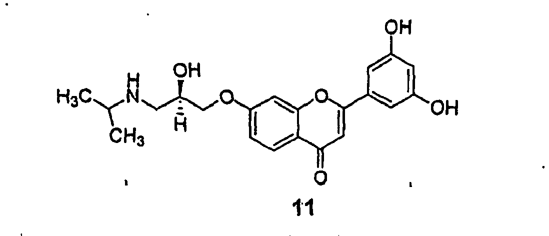

Still another aspect of the present disclosure provides a compound having formula I or a pharmaceutically acceptable salt thereof, wherein the representative compounds are:
a) R-(-)-7-(3-tert-Butylamino-2-hydroxy-propoxy)-3', 5'-dibenzyloxy-flavone (4);
b) S-(+) - 7- (3-tert-Butylamino-2-hydroxy-propoxy)-3', 5'-dibenzyloxy-flavone (7)
c) R-(-)-7-(3-iso-propylamino-2-hydroxy-propoxy) - 3', 5'-dibenzyloxy - flavone (8);
d) R-(-)-7-(3-iso-propylamino-2-hydroxy-propoxy) - 3', 5'-dihydroxy - flavone (9);
e) S-(+)-7-(3-iso-Propylamino-2-hydroxy-propoxy) - 3', 5'-dibenzyloxy- flavone (10);
f) S-(+)-7-(3-iso-Propylamino-2-hydroxy-propoxy) - 3', 5'-dihydroxy- flavone (11).

Accordingly the present invention provides a process for preparation of compound of general formula I, comprising the steps of:
(i) reacting substituted hydroxyflavone of general formula A wherein R₁, R₂ and R₃ are selected from the group consisting of hydrogen, methyl or iso pentenyl; wherein R₄, R₅ and R₆ are selected from the group consisting of O-alkyl, O-benzyl, O-substituted hydroxy, phenyl or combination thereof, with optically active **R** or **S** *epi*chlorohydrin followed by reaction with a base to obtain the inverted 2,3-epoxy-propoxy-flavones of compound of formula **B,** 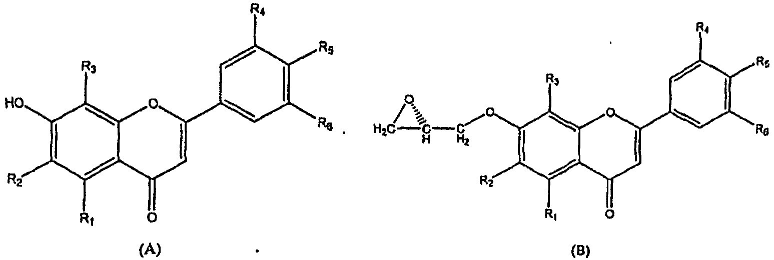
(ii) heating the 2,3-epoxy-propoxy-flavones of the formula **B** as obtained in step (i) under reflux, with an amine in an alcohol to yield propanolamines of formula C wherein R' is selected from a group consisting of t-butyl amine, n-butylamine, isobutylamine, iso-propyl amine, 4-phenyl-piperazine-1-ylamine, 4-(2-methoxyphenyl)-piperazin-1-ylamine, and 3, 4-dimethoxy phenethyl amine, 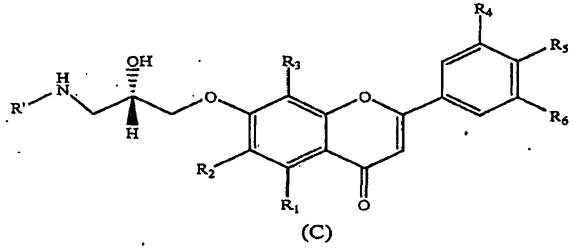
(iii) subjecting the propanolamine obtained in step (ii) for debenzylation using catalytic hydrogenation process to yield corresponding hydroxy flavone derived propanolamines of general formula C wherein R₄, R₅, and R₆ are selected from the group of H, OH and O-methoxy. 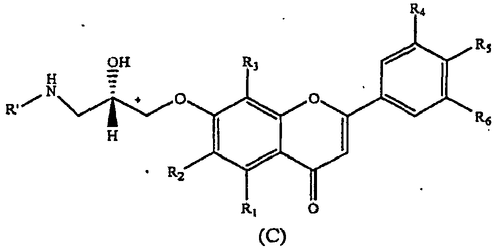
(iv) converting the compound of formula C into salts by known method.

In an embodiment of the invention, the base is selected from the group consisting of sodium hydroxide, potassium hydroxide and benzyltriethylammonium hydroxide.

In another embodiment of the invention, the amine used is selected from the group consisting of alkylamines from a group of cyclic and acyclic alkyl part.

In another embodiment of the invention, the alcohol used is selected from the group consisting of ethanol and propanol.

In another embodiment of the invention, the debenzylation is carried out using Pd-C as catalyst under hydrogen pressure ranging between 40-100 psi (276-690 kPa).

In another embodiment of the invention, the salt of general formula is selected from the group consisting of hydrochloride, fumarate and tartrates

Accordingly the present disclosure provides a method of treating type II diabetes and dislipidemia in mammals, said method comprising the step of administering pharmaceutically effective amount of compound of formula I, and salts thereof, optionally with the pharmaceutically acceptable exciepients.

In an aspect of the disclosure, the pharmaceutically effective amount of compound of formula I is in the range of 10-100 mg. /kg-body weight.

In another aspect of the disclosure, the subject may be selected from animals including human.

In another embodiment of the invention, the compound of formula I showed maximum fall in blood glucose was recorded to be around 61.1% at a dose around 25 mg/kg body weight.

In a further embodiment of the invention, the compound (S)-(+)-7-(3-*tert-*butylamino-2-hydroxypropoxy)-3', 5'-dibenzyloxyflavone (7) showed an elevation of 33.2% was measured in HDL-cholesterol level than the control ones at dose around 25 mg/kg body weight.

In yet another embodiment of the invention, ED₅₀ of compound of general formula I is in the range of 17.84 to 32.93 mg/kg.

In another embodiment of the invention, the compound (S)-(+)-7-(3-*tert-*butylamino-2-hydroxypropoxy)-3', 5'-dibenzyloxyflavone (7) enhances HDL-C more than two folds as compared to the racemic compound at a dose of 25mg/kg body weight.

### Chemistry:

Aryloxypropanolamines are well established pharmacophore for β-adrenergic receptors and therefore various enantioselective syntheses for example, using chiral glycerol-1, 2-acetonide [Danilewicz J.C., Kemp .J.E.G., J. Med. Chem., 16168 (1973)], glycidylarenesulfonate [Klunder J.M., Onami T. Sharpless K.B., J. Org. Chem 54, 1295 (1989)] or *epi*-chlorohydrin [Baldwin J.J., Raab A.W., Mensler K., Arison B.H.., McClure D. E., J.Org. Chem. 43, 4876 (1978)] and [Seki T., Kanada A., Nakao T., Shiraiwa M., Asano H., Miyazawa K., Ishimori T., Minami N., Shibata K., Yasuda K., Chem Pharma Bull 46(1), 84 (1998)] are known in literature. A phenoxide anion reacts with glycidyl derivative either at C-1 or C-3 depending on the reactivity substituent at allylic position of oxyrane. The attack at C-1 causes direct displacement of X (path a) in case of arenesulfonate whereas that at C-3 in case of *epi*-chlorohydrin causes epoxide ring opening, which following internal displacement of the leaving group X (path b) as shown in Fig I results the product of opposite configuration to path a. We synthesized the optical isomers of our product following path b.

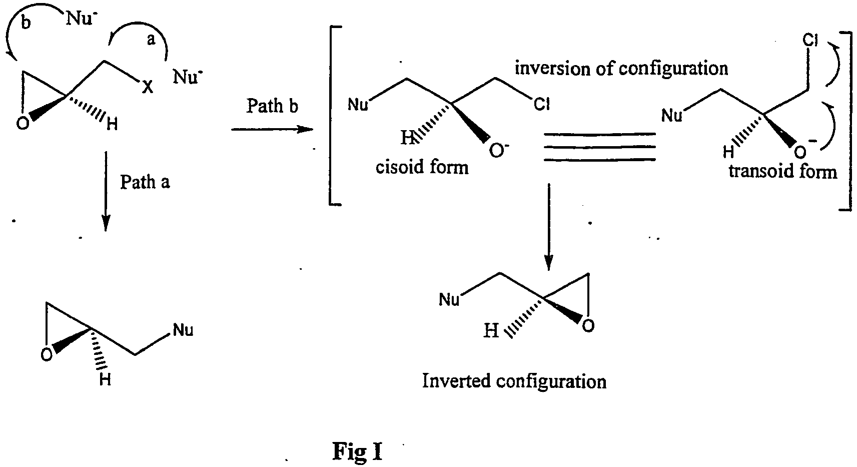

Therefore when the reaction of flavone was carried out with (R)-*epi*-chlorohydrin at 120°C without the solvent, the intermediate chlorohydrin was obtained in an excellent yield. The chlorohydrin without purification taken in toluene and treated with aqueous NaOH in presence of catalytic amount of phase transfer catalyst triethylbenzylammonium chloride (TEBA) to give S-isomer in 79% yield with 95% ee. Thus the nucleophilic substitution reaction at 120°C proceeded at the terminal C atom of the epoxide ring with high regioselectivity, probably via activating complexation (Fig II) as presumed by McClure D.E., Arison B.H., Baldwin J.J., J. Am. Chem. Soc., 101, 3666(1979).

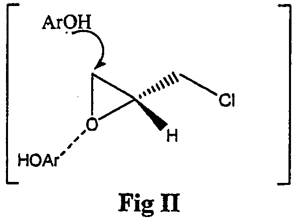

The epoxide thus obtained was treated with appropriate amine in methanol to yield desired amino propanol derivative. The purity of these compounds was ascertained by HPLC on comparison of the racemic mixture graph with that of the enantiomers graph. The HPLC study was performed on Chira Sphere NT (250 mm x 0.4 mm, 5µm, Merck) column using a mixture of solvent hexane: iso-propanol: methanol: ammonia (in a ratio of 60:40:5:0.5 ml) at the flow rate of 2ml/min and peak detection at 240 nm under UV. The graph of one of the mixtures and its enantiomers are given in figure III.

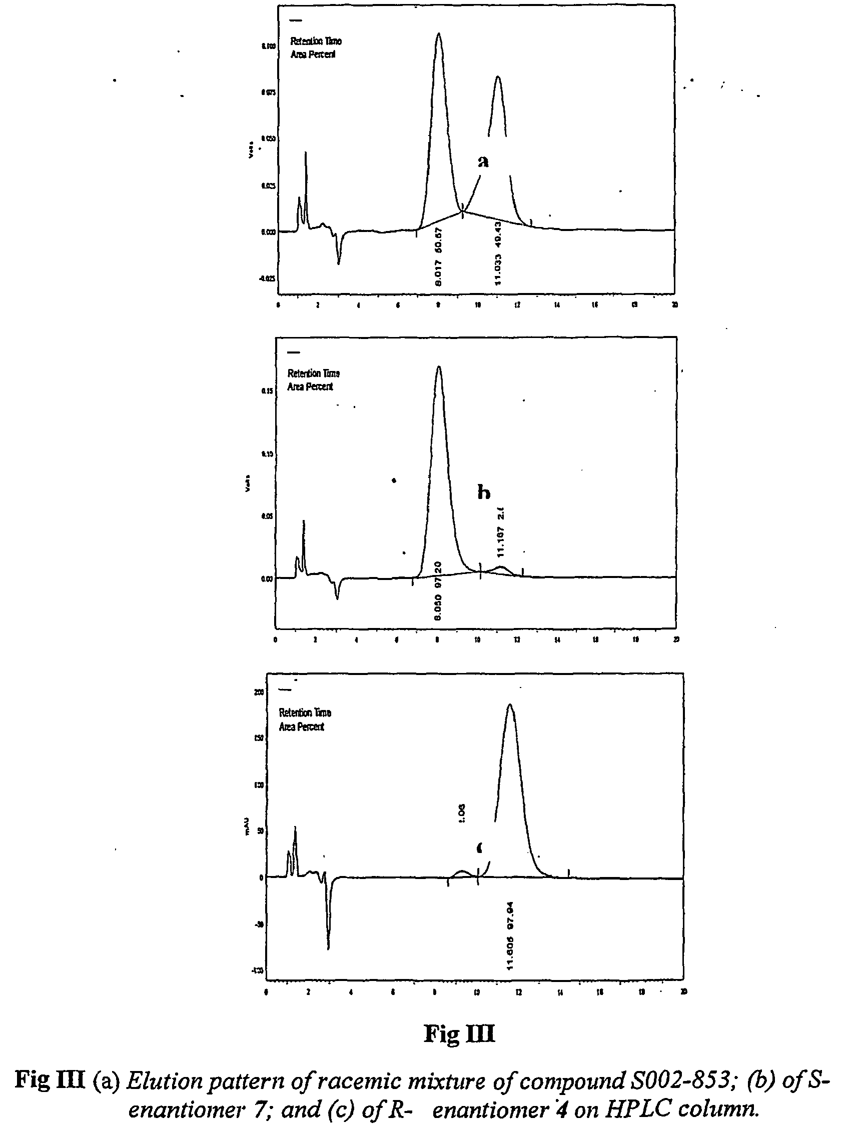

### Comparative example 1

### (R)- (-)-7-(3-tert-Butylamino-2-hydroxypropoxy)-3',5'-dibenzyloxyflavone (4, S002-853 R, Fig IV):

A mixture of 3', 5'-dibenzyloxy-7-hydroxy flavone (1, 1.0 gm, 2.2 mmol) and (S)-*epi*-chlorohydrin (1.0 ml, 12.0 mmol) was stirred for about 2 hrs at 120°C. The cooled reaction mixture was taken in toluene and to this was added dropwise a solution of NaOH (10 mg, 0.25 mmol) and benzyltriethyl ammonium chloride (10 mg, 0.04 mmol) in water with stirring at room temperature for 2 hrs, and the organic layer was separated, dried over sodium sulphate and solvent evaporated under reduced pressure to give crude 3', 5'-dibenzyloxy-7-(2, 3-epoxy-propoxy) flavone (3) of R- configuration.
Yield (900 mg, 80%)

The above (R)-3', 5'-dibenzyloxy -7-(2, 3-epoxy-propoxy) flavone (600 mg, 4.9 mmol) and *tert*-butyl amine (0.26 ml, 9.8 mmol) in dry methanol (50 ml) was stirred at reflux for about 6 hrs. The solvent was evaporated at reduced pressure and the crude product was purified by column chomatography to afford (R)-(-) 7-(3-*tert*- butylamino-2-hydroxypropoxy)-3', 5'-dibenzyloxyflavone (4) Yield (650 mg, 93%); m.p 158°C; [α]_{D}=- 8.6 (c=1.5%, CHCl₃).

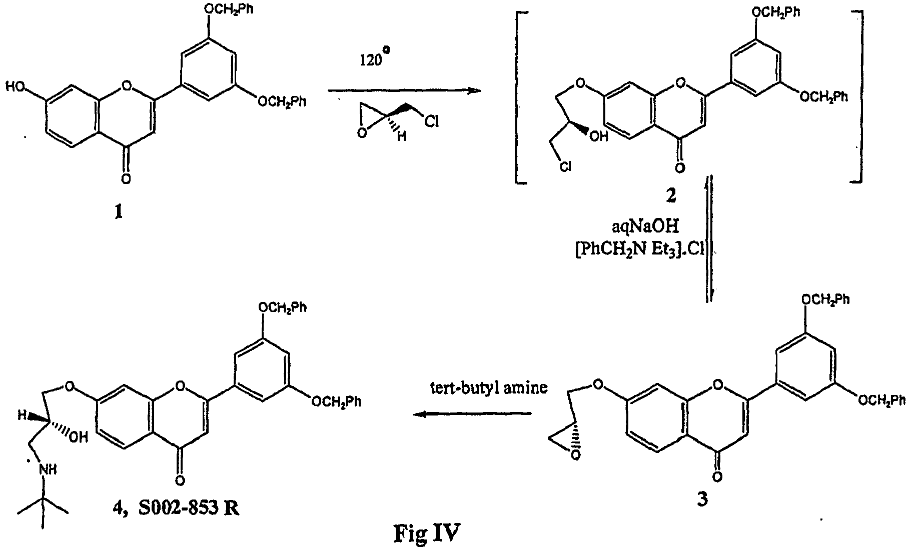

### Example 1

### (S)- (+)-7-(3-tert-Butylamino-2-hydroxypropoxy)-3', 5'-dibenzyloxyflavone (7, S002-853 S, Fig V):

A mixture of 3', 5 '-dibenzyloxy-7-hydroxy flavone (1) (1.2 g, 2.6 mmol) and (R)-*epi*- chlorohydrin (1.12 ml, 15.0 mmol) was stirred for about 2 hrs at 120°C. The cooled reaction mixture was taken in toluene and to this was added a solution of NaOH (15mg., 0.37mmol) and benzyltriethylammoniumchloride (15 mg, 0.06 mmol) in.water, stirred at room temperature for 2 hrs. The organic layer was separated, dried over sodium sulphate and solvent removed under reduced pressure to give crude (S)-(+)-7-(2, 3-epoxy-propoxy)- 3', 5'-dibenzyloxy flavone (6). Yield (1.12 g, 83%)

The solution of above (S)-3', 5'-dibenzyloxy -7-(2, 3-epoxy-propoxy)-flavone (500 mg, 4.9 mmol) in dry methanol (50 ml) and *tert-*butyl amine (0.2 ml,9.8 mmol) was stirred at reflux for about 6 hrs. The solvent removed at reduced pressure and crude product was purified by column chromatography to afford (S)-(+)-7-(3-*tert*-butylamino-2-hydroxy-propoxy)-3' 5'-dibenzyloxy-flavone (7). Yield (520 mg, 93%); m.p165°; [α]_{D}= +9.0 (c=20 mg/7.5 ml of CHCl₃).

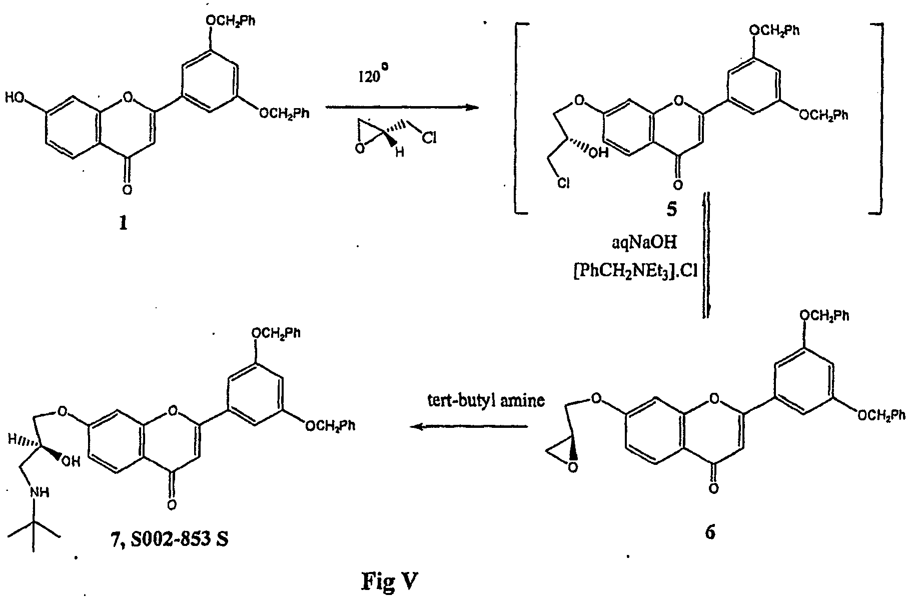

### Comparative example 2

### (R)- (-)- 7-(2-Hydroxy-3-isopropyl aminopropoxy) - 3', 5'-dihydroxy flavone (9, S002-857 R, Fig VI):

A mixture of 3', 5'-dibenzyloxy-7-hydroxy-flavone (1) (1 g, 2.2 mmol) and S-epi-chlorohydrin (1.0 ml, 12.0 mmol) was stirred for about 2 hrs at 120 °C. The cooled mixture was taken in toluene and to this was added a solution of NaOH (10 mg, 0.25 mmol) and benzyltriethyl ammonium chloride (10 mg, 0.04 mmol) in water and stirred at room temperature for 2 hrs. The organic layer was separated, dried over sodium sulphate and evaporated under reduced pressure to give crude R-3', 5'-dibenzyloxy-7-(2, 3-epoxy-propoxy)-flavone (3). Yield (900 mg, 80%)

The solution of above (R)-3', 5'-dibenzyloxy-7-(2,3-epoxy-propoxy)-flavone (600 mg,4.9 mmol) and *iso*- propylamine (0.5 ml,14.7 mmol) in dry methanol (50 ml) was stirred at reflux for about 6 hrs. The solvent was removed at reduced pressure and the crude product so obtained was purified by column chomatography to give (R) - 7-(2-hydroxy-3-isopropyl amino-propoxy)-3', 5'-dibenzyloxy flavone **(8)** Yield (550 mg, 79%)

(R)-3', 5'-Dibenzyloxy-7-(2-hydroxy-3-*iso*propyl amino-propoxy) flavone (300 mg, 2.12 mmol) was shaken in dry methanol (40 ml) with 10 % Pd/C (quantity sufficient) under hydrogen atmosphere. The catalyst was filtered and the solvent removed under reduced pressure to give the product (R) -(+)-7-(2-hydroxy-3-*iso*propylamino-propoxy)- 3', 5'-dihydroxy flavone (9), Yield (200 mg, 98%) m.p; 124°C; [α]_{D} = -9 (c=20 mg/ 7.5 ml of 2% CHCl₃/MeOH).

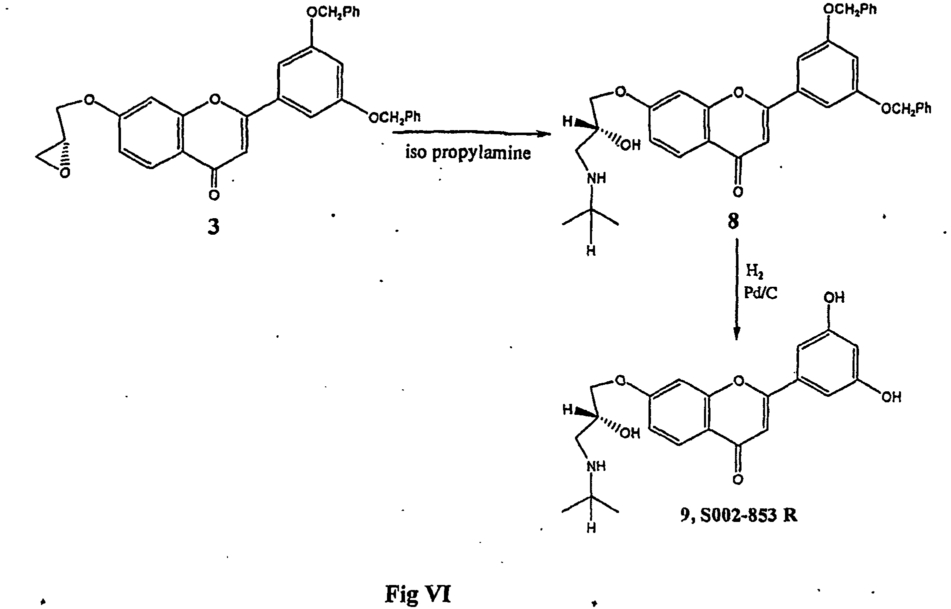

### Example 2

### (S)- (+) -7-(2-Hydroxy-3-isopropyl aminopropoxy) - 3', 5'-dihydroxyflavone (11, S002-857 S, Fig VII):

A mixture of 3', 5'-dibenzyloxy-7-hydroxyflavone (1.2 g, 2.6 mmol) and *R-epi-*chlorohydrin (1.2 ml, 15.0 mmol) was stirred for about 2 hrs at 120°. The cooled reaction mixture was taken in toluene and to this was added a solution of NaOH (15 mg,0.37 mol) and benzyltriethyl ammonium chloride (15 mg,0.06 mmol) in water and stirred at room temperature for 2 hrs. The organic layer was separated, dried over sodium sulphate and solvent removed under reduced pressure to give crude S-3', 5'-dibenzyloxy-7-(2, 3-epoxy-propoxy) flavone (6) Yield (1.12 g, 83%).

The solution of above (S) -7-(2, 3-epoxy-propoxy)-3',5'-dibenzyloxy- flavone (500 mg, 4.9 mmol) and *iso*-propylamine(0.3 ml, 14.7 mmol) in dry methanol (50 ml) was stirred at reflux for about 6 hrs. The solvent removed at reduced at reduced pressure the crude product so obtained was purified by column chomatography to afford (S) - 7-(2-hydroxy-3-*iso*propyl amino-propoxy)-3', 5'-dibenzyloxy- flavone (10) Yield (440 mg,79%).

(S)-7-(2-Hydroxy-3-*iso*propylamino-propoxy)-3',5'-dibenzyloxy-flavone (250 mg, 2.12 mmol) in dry methanol (40 ml) and 10% Pd/C (sufficient amount) was shaken in hydrogen atmosphere at about 40 psi (276 kPa). The catalyst was filtered and solvent removed to give the product (S)-(+) -7-(2-hydroxy-3-*iso-*propylamino-propoxy) - 3', 5'-dihydroxy-flavone (11) Yield (160 mg, 98%); m.p.130°C; [α]_{D}=+15.4 (c=20 mg/ 7.5 ml of 2% CHCl₃/MeOH).

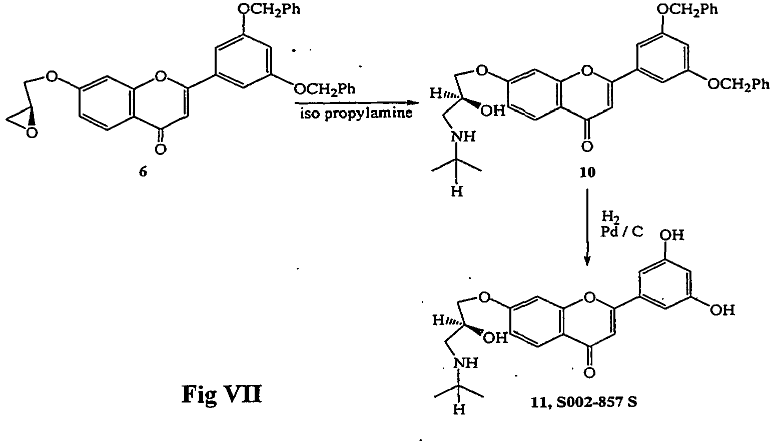

### Biological Evaluation:

### Experimental Protocol:

C57BL/KsBom-db/db mice (12 - 18 weeks old), weighing around 35 + g bred in the animal house of CDRI, Lucknow, were used in the present experiments. The mice were housed in groups of 3 to 5 (same sex) in a room controlled for temperature (23 ± 2.0 °C) and 12/12 hours light/dark cycle (lights on at 6.00 am). Body weight of each animal was measured daily from day 1 to day 10 of the experiment. All animals had free access to fresh water and normal pellet diet except on the day of the postprandial protocol (day 6) and during the overnight fast before the OGTT on day 10. The animals always had access to water during experimental periods. Blood glucose was checked every morning up till day 10. On day 10 an oral glucose tolerance test (OGTT) was performed after an overnight fasting. Blood glucose was measured at -30.0 min and test compounds were administered. The blood glucose was again measured at 0 min just post treatment and at this juncture glucose solution was given at a dose of 3 gm/kg to all the groups including the vehicle treated group. The blood glucose levels were checked at 30 min, 60 min, 90 min and 120 min post glucose administration. The data was analyzed for its significance on Prism software.

### Antihyperglycemic Activity Profile:

### (S)- (+)-7-(3-tert-Butylamino-2-hydroxypropoxy) -3', 5'-dibenzyloxyflavone (7, S-002-853 S):

It is evident from the data that compound **(7, S-002-853 S)** exhibit significant antihyperglycemic and antidyslipidemic activity in db/db mice at 25 mg/kg dose. At this dose it showed maximum fall in blood glucose profile at day 6 of treatment as shown in the table 1. The maximum percentage fall in blood glucose was recorded to be around 61.1%. Fig VIII shows the graphical presentation of blood glucose profile versus days of treatment at 25 mg/kg dose level. Fig IX and table 2 presents effect of the 10 days treatment of the compound on glucose tolerance of the db/db mice. This effect was calculated to be around 45.3 %.

Table 3 and Fig X presents the antidyslipidemic activity profile of (S)-(+)-7-(3-*tert*-butylamino-2-hydroxy propoxy)-3', 5'-dibenzyloxyflavone **(7, S-002-853 S)** in db/db mice at 25 mg/kg dose level. A lowering in the case of serum cholesterol and triglycerides by the treatment of (S)-(+)-7-(3-*tert*-butylamino-2-hydroxylpropoxy)-3', 5'-dibenzyloxyflavone **(7, S-002-853 S)** was recorded which was around 21.8% and 14.5%, respectively, at 25 mg/kg dose. An elevation of 33.2% was measured in HDL-cholesterol level than the control ones while a decrease of around 29.4% was measured in LDL-cholesterol level in (S)-(+)-7-(3-*tert*-butylamino-2-hydroxypropoxy)-3', 5'-dibenzyloxyflavone **(7, S-002-853 S)** treated db/db mice at 25 mg/kg dose.

**Table 1: Blood glucose profile of sham treated and (S)-(+)-7-(3-tert-butylamino-2-hydroxypropoxy)-3', 5'-dibenzyloxyflavone (7, S-002-853 S) treated db/db mice.**

| | **Blood glucose (mM) days post treatment** | | | | | | | | |
|---|---|---|---|---|---|---|---|---|---|
| | **1** | **2** | **3** | **4** | **5** | **6** | **7** | **8** | **9** |
| **Sham treated** | 22.4 ±1.69 | 25.0 ±3.24 | 21.4 ±5.07 | 23.6 ±3.81 | 19.8 ±4.63 | 19.4 ±5.12 | 22.2 ±5.91 | 20.8 ±4.80 | 19.1 ±4.10 |
| **S-002-853 (S) treated (25 mg/kg)** | 22.4 ±1.24 | 16.6 ±3.85 | 12.9 ±3.00 | 10.6 ±2.89 | 8.80 ±3.84 | 7.60 ±3.46 | 10.6 ±2.20 | 8.30 ±1.13 | 7.90 ±1.30 |
| **% change compared to control** | **+0.20** | **-33.6** | **-39.5** | **-54.9** | **-55.6** | **-61.1** | **-52.2** | **-60.0** | **-58.5** |
| **Statistical significance** | **ns** | **ns** | **ns** | **p<0.05** | **P<0.05** | **p<0.05** | **p<0.05** | **p<0.05** | **p<0.05** |

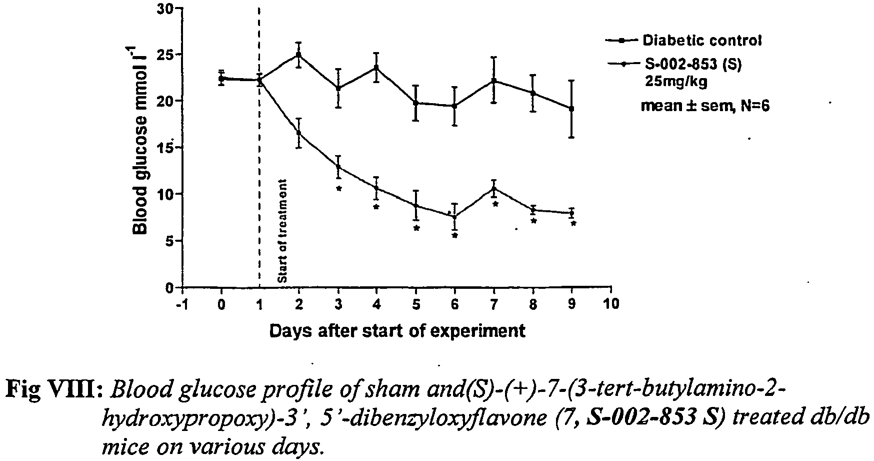

**Table 2: Blood glucose profile of sham treated and (S)-(+)-7-(3-tert-butylamino-2-hydroxypropoxy)-3',5'-dibenzyloxyflavone (7, S-002-853 S) treated db/db mice.**

| | **Blood glucose profile (mM) min post glucose load** | | | | | |
|---|---|---|---|---|---|---|
| | **-30** | **0** | **30** | **60** | **90** | **120** |
| **Sham treated** | 8.09 ±0.63 | 8.28 ±0.63 | 29.0 ±2.06 | 31.5 ±0.91 | 23.8 ±2.73 | 21.1 ±3.36 |
| **S-002-853 S treated (25 mg/kg)** | 4.57 ±0.30 | 4.81 ±0.35 | 17.8 ±1.24 | 16.3 ±1.82 | 13.0 ±1.66 | 8.83 ±0.72 |
| **% change compared to control** | **-43.5** | **-41.8** | **-38.6** | **-48.3** | **-45.5** | **-58.2** |

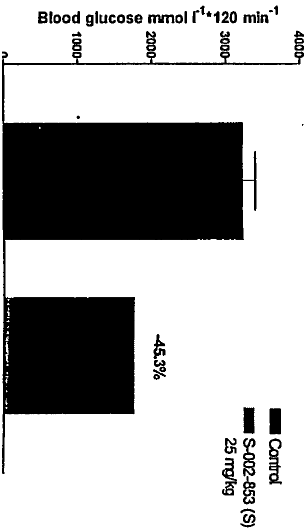

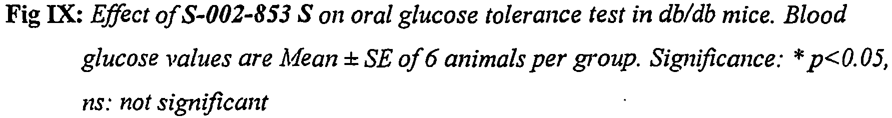

**Table 3: Serum lipid profile in sham treated and (S)-(+)-7-(3-tert-butylamino-2-hydroxypropoxy)-3',5'-dibenzyloxyflavone (7, S-002-853 S) treated db/db mice.**

| | **Serum lipid profile (mg/dl)** | | |
|---|---|---|---|
| | **Triglycerides** | **Cholesterol** | **HDL-Chol** |
| **Sham treated** | 135±3.32 | 238±24.6 | 37.2±1.92 |
| **S-002-853 S (25 mg/kg)** | 115±2.82 | 185±51.4 | 49.5±8.36 |
| **% change compared to control** | **-14.5** | **-21.8** | **+33.2** |

| | | | |
|---|---|---|---|
| - denotes decrease + denotes increase Values are Mean ±SE of 6 animals per group | | | |

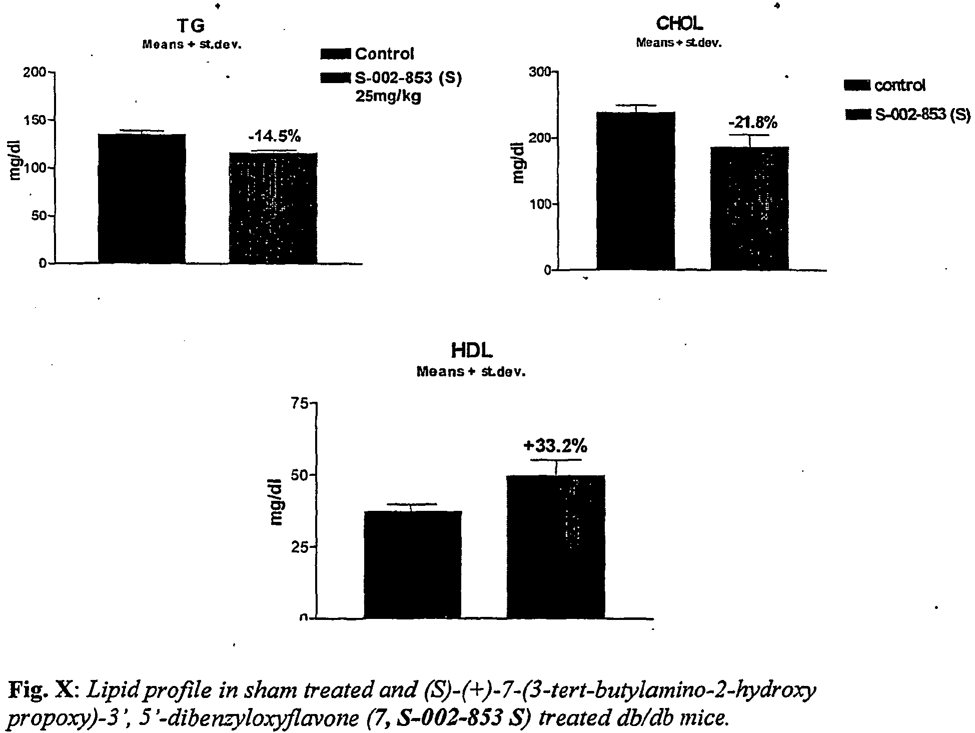

Tables 4 and 6 presents antihyperglycemic and antidyslipidemic activity profile of the db/db mice treated with (S)-(+)-7-(3-*tert*-butylamino-2-hydroxypropoxy)-3',5'-dibenzyloxyflavone (7, S-002-853 S) at 10 mg/kg dose. Though, at this dose level it showed fall in blood glucose profile from day 3 of treatment and remained till the end of study. However, it was not statistically significant on any day post treatment. Fig XI shows the graphical presentation of blood glucose profile versus days of treatment at 10mg/kg dose level. Table 5 and Fig XII presents effect of the 10 days treatment of the compound on glucose tolerance of the db/db mice. This effect was calculated to be around 27.6% but was not statistically significant. Table 6 and fig XIII presents the antidyslipidemic activity profile of (S)-(+)-7-(3-*tert*-butylamino-2-hydroxypropoxy)-3', 5'-dibenzyloxyflavone (**7, S-002-853 S**) in db/db mice at 10 mg/kg dose level. A lowering in the case of serum cholesterol and triglycerides by the treatment of (S)-(+)-7-(3-*tert*-butylamino-2-hydroxypropoxy)-3', 5'-dibenzyloxyflavone **(7, S-002-853 S)** was recorded which was around 16.3% and 17.9%, respectively, at 10 mg/kg dose. An elevation of +17.4% was measured in HDL-cholesterol level than the control ones. These difference, however, were statistically insignificant.

**Table 4: Blood glucose profile of sham treated and (S)-(+)-7-(3-tert-butylamino-2-hydroxypropoxy)-3',5'-dibenzyloxyflavone (7, S-002-853 S) treated db/db mice.**

| | **Blood glucose (mM) days post treatment** | | | | | | | | |
|---|---|---|---|---|---|---|---|---|---|
| | **1** | **2** | **3** | **4** | **5** | **6** | **7** | **8** | **9** |
| **Sham treated** | 19.7 ±2.11 | 20.4 ±2.84 | 21.9 ±2.66 | 21.3 ±2.21 | 22.1 ±1.36 | 20.4 ±3.55 | 20.8 ±3.82 | 23.5 ±3.88 | 12.4 ±2.04 |
| **S-002-853 (S) treated (10 mg/kg)** | 19.8 ±2.47 | 18.5 ±2.34 | 17.9 ±2.14 | 15.3 ±2.96 | 13.8 ±2.98 | 13.1 ±3.41 | 11.8 ±3.55 | 12.1 ±3.39 | 14.2 ±2.98 |
| **% change compared to control** | **-0.9** | **-9.60** | **-18.6** | **-26.8** | **-35.0** | **-40.7** | **-42.0** | **-42.2** | **-39.4** |
| **Statistical significance** | **ns** | **ns** | **ns** | **ns** | **ns** | **ns** | **ns** | **ns** | **ns** |

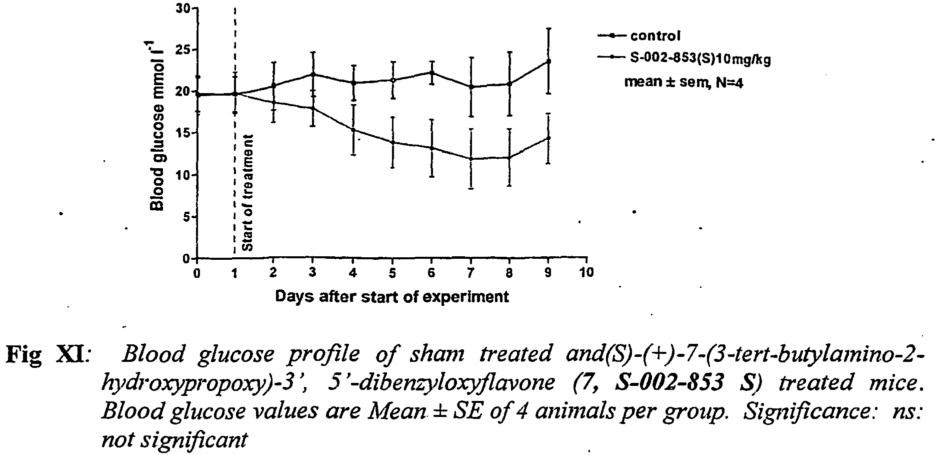

**Table 5: Blood glucose profile of sham and (S)-(+)-7-(3-tert-butylamino-2-hydroxypropoxy)-3',5'-dibenzyloxyflavone (7, S-002-853 S) treated db/db mice post oral glucose load.**

| | **Blood glucose profile (mM) min post glucose load** | | | | | |
|---|---|---|---|---|---|---|
| | **-30** | **0** | **30** | **60** | **90** | **120** |
| **Sham treated** | 11.0 ±2.02 | 12.4 ±2.04 | 28.7 ±2.67 | 25.3 ±3.89 | 16.6 ±2.45 | 10.4 ±0.43 |
| **S-002-853 (S) treated (10 mg/kg)** | 8.37 ±2.98 | 9.23 ±3.10 | 19.3 ±5.52 | 18.4 ±5.39 | 12.1 ±3.81 | 9.23 ±3.10 |
| **% change compared to control** | **-23.9** | **-25.7** | **-32.8** | **-27.2** | **-26.9** | **-11.7** |

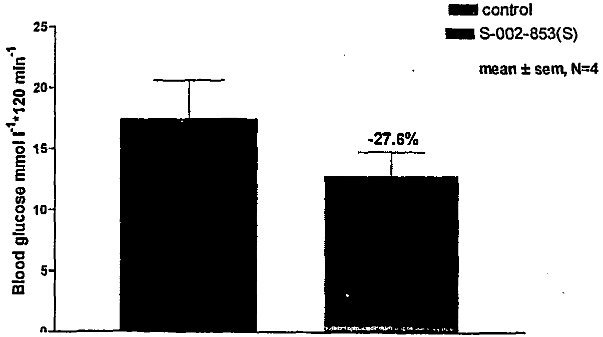

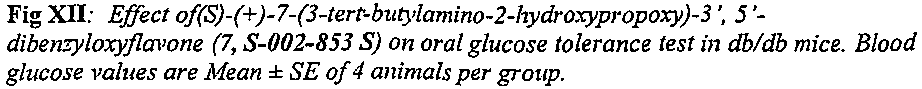

**Table 6: Serum lipid profile in sham treated and (S)-(+)-7-(3-tert-butylamino-2-hydroxypropoxy)-3',5'-dibenzyloxyflavone (7, S-002-853 S) treated db/db mice.**

| | **Serum lipid profile (mg/dl)** | | |
|---|---|---|---|
| | **Triglycerides** | **Cholesterol** | **HDL-Chol** |
| **Sham treated** | 134±12.7 | 104±8.27 | 56.0±2.45 |
| **S-002-853 (S) (10 mg/kg)** | 110±4.08 | 87.5±3.00 | 66.4±2.87 |
| **% change compared to control** | **-17.9** | **-16.3** | **+17.4** |

| | | | |
|---|---|---|---|
| - denotes decrease + denotes increase Values are Mean ±SE of 4 animals per group | | | |

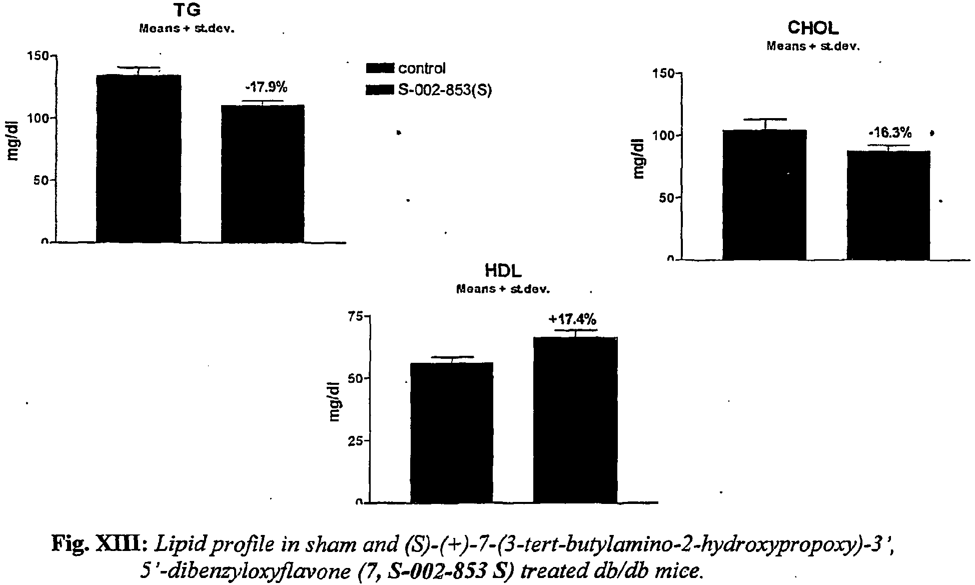

### (R)- (-)-7-(3-tert-Butylamino-2-hydroxypropoxy)-3',5'-dibenzyloxyflavone (4, S-002-853 R):

Tables 7 and 9 present antihyperglycemic and antidyslipidemic activity profile of the db/db mice treated with **(4, S-002-853 R)** at 25 mg/kg dose. At this dose level, though the blood glucose profile of S-002-853 (R) treated mice was lowered from day 3 and it remained the same through the period of study. However, there was found no significant difference in the blood glucose profile between the two groups at any day. Fig XIV shows the graphical presentation of blood glucose profile versus days of treatment at 25 mg/kg dose level. There was also found no difference in oral glucose tolerance curve between the sham treated and S-002-853 (R) treated db/db mice. (Table 8; Figure XV). Table 9 presents the lipid profile of **S-002-853 R and sham treated groups.** There was found no significant lowering in either the case of serum cholesterol or triglycerides by the treatment of (R)-(-)-7-(3-*tert*-butylamino-2-hydroxypropoxy)-3',5'-dibenzyloxyflavone **(4, S-002-853, R).** There was also found no significant difference in the HDL-cholesterol levels between the sham and S-002-853 (R) treated groups (Table 9, fig XVI).

**Table 7: Blood glucose profile of sham treated and (R)-(-)-7-(3-tert-butylamino-2-hydroxypropoxy)-3',5'-dibenzyloxyflavone (4, S-002-853 R) treated db/db mice.**

| | **Blood glucose (mM) days post treatment** | | | | | | | | |
|---|---|---|---|---|---|---|---|---|---|
| | **1** | **2** | **3** | **4** | **5** | **6** | **7** | **8** | **9** |
| **Sham treated** | 11.8 ±3.31 | 12.2 ±3.56 | 15.1 ±5.26 | 17.5 ±2.08 | 15.0 ±0.45 | 15.9 ±0.46 | 18.1 ±1.62 | 19.7 ±0.75 | 20.8 ±2.06 |
| **S-002-853 (R) treated (25 mg/kg)** | 12.0 ±2.52 | 12.4 ±2.65 | 10.3 ±1.33 | 13.3 ±4.61 | 12.3 ±4.32 | 12.1 ±3.82 | 14.5 ±5.49 | 13.4 ±4.54 | 13.5 ±4.66 |
| **% change compared to control** | **+1.1** | **-31.9** | **-24.1** | **-18.1** | **-24.0** | **-19.8** | **-31.9** | **-35.4** | **-38.3** |
| **Statistical significance** | **ns** | **ns** | **ns** | **ns** | **ns** | **ns** | **ns** | **ns** | **ns** |

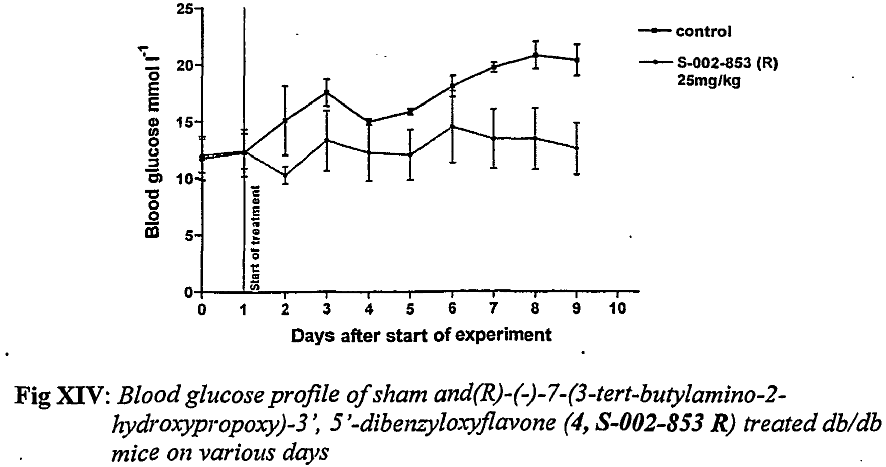

**Table 8: Blood glucose profile of sham and (R)-(-)-7-(3-tert-butylamino-2-hydroxypropoxy)-3',5'-dibenzyloxyflavone (4,S-002-853 R) treated db/db mice post oral glucose load.**

| | **Blood glucose profile (mM) min post glucose load** | | | | | |
|---|---|---|---|---|---|---|
| | **-30** | **0** | **30** | **60** | **90** | **120** |
| **Sham treated** | 16.5 ±8.08 | 21.0 ±5.81 | 33.1 ±0.45 | 25.9 ±0.31 | 21.0 ±2.68 | 18.4 ±2.39 |
| **S-002-853 (R) treated (25 mg/kg)** | 13.6 ±0.46 | 20.3 ±4.74 | 34.3 ±16.9 | 25.9 ±9.75 | 24.5 ±9.13 | 23.0 ±10.0 |
| **% change compared to control** | **-17.4** | **-3.30** | **+3.70** | **0.00** | **+17.0** | **+24.9** |

| | | | | | | |
|---|---|---|---|---|---|---|
| Blood glucose values are Mean ± SE of 3 animals per group. Significance: ns: not significant | | | | | | |

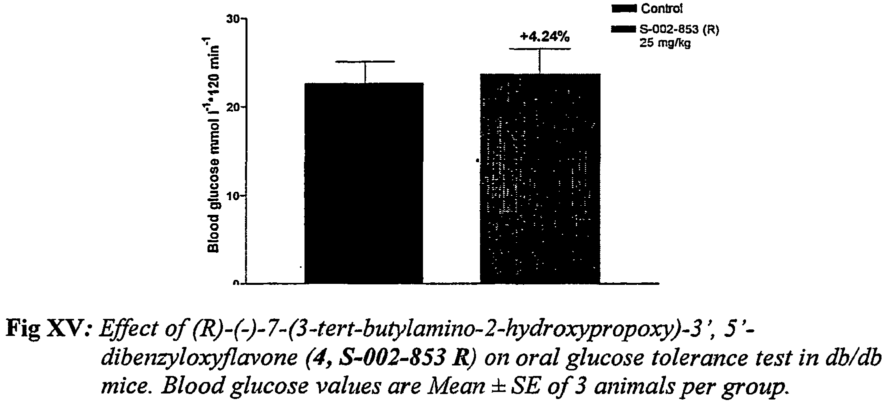

**Table 9: Serum lipid profile in sham and (R)-(-)-7-(3-tert-butylamino-2-hydroxypropoxy)-3',5'-dibenzyloxyflavone (4, S-002-853 R) treated db/db mice.**

| | **Serum lipid profile (mg/dl)** | | |
|---|---|---|---|
| | **Triglycerides** | **Cholesterol** | **HDL-Chol** |
| **Sham treated** | 147.0±30.6 | 150.0±4.51 | 53.6±4.93 |
| **S-002-853 (R) treated (25 mg/kg)** | 132.0±11.5 | 144.0±7.37 | 58.0±6.24 |
| **% change compared to control** | **-9.72** | **-3.72** | **+8.06** |

| | | | |
|---|---|---|---|
| Values are Mean ± SE of 3 animals per group | | | |

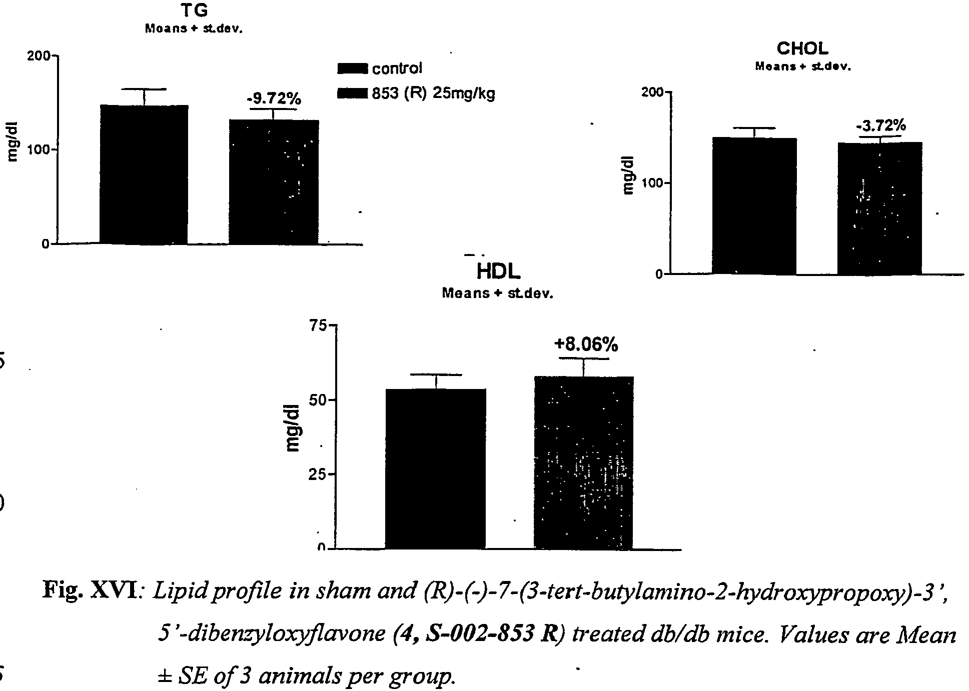

### (S)- (+)-7-(2-Hydroxy-3-isopropylaminopropoxy)-3',5'-dihydroxyflavone(11,S-002-857S):

Table 10 presents the blood glucose profile of sham and S-002-857 S treated db/db mice. It is evident from the results that S-002-857 (S) treatment lowered the blood glucose profile of db/db mice. It is evident from the results that 25.0 mg/kg S-002-857 (S) treatment lowered the blood glucose profile from day 4th and continued till the end. An average blood glucose lowering was calculated to be around 20 % in this case with S-002-857 (S). The lowering in blood glucose was statistically significant from day 7 to 9 where around 37.4, 36.8 and 43.3 % effect was observed. Figure XVII is the graphical presentation of blood glucose profile versus days of treatment at 25 mg/kg dose level. Table 11 and figure XVIII presents the oral glucose tolerance curves of sham treated and S-002-857 (S) treated db/db mice. An overall improvement of 27 % was evident in the case with S-002-857 (S) at 25.0 mg/kg compared to sham treated group

Table 12 and figure XIX presents the lipid profile of sham and S-002-857 S treated db/db mice. It is evident from the result that 25.0 mg/kg S-002-857 S) treated db/db mice had though lowered serum cholesterol, triglycerides profile compared to sham treated db/db mice but these profiles were not statistically significant. However, an elevation of 17.3% was noted in HDL-cholesterol level in S-002-857 (S) treated db/db mice compared to the sham treated controls.

**Table 10: Blood glucose profile of sham treated and (S)-(+) -7-(2-Hydroxy-3-isopropyl aminopropoxy)- 3', 5'-dihydroxy flavone (11, S-002-857 S) treated db/db mice.**

| | **Blood glucose (mM) days post treatment** | | | | | | | | |
|---|---|---|---|---|---|---|---|---|---|
| | **1** | **2** | **3** | **4** | **5** | **6** | **7** | **8** | **9** |
| **Sham treated** | 12.5 ±1.94 | 12.4 ±1.90 | 12.5 ±1.97 | 13.0 ±2.40 | 13.6 ±3.43 | 13.0 ±1.65 | 15.0 ±1.39 | 14.7 ±2.69 | 13.3 ±3.18 |
| **S-002-857 (S) treated (25 mg/kg)** | 12.7 ±2.61 | 12.3 ±2.40 | 11.5 ±1.80 | 11.3 ±2.54 | 10.1 ±2.08 | 9.19 ±2.25 | 9.41 ±1.37 | 9.28 ±1.02 | 7.56 ±2.13 |
| **% change compared to control** | **-1.60** | **-0.80** | **-8.13** | **-12.7** | **-25.3** | **-29.1** | **-37.4** | **-36.8** | **-43.3** |
| **Statistical significance** | **ns** | **ns** | **ns** | **ns** | **ns** | **ns** | **p<0.05** | **p<0.05** | **p<0.05** |

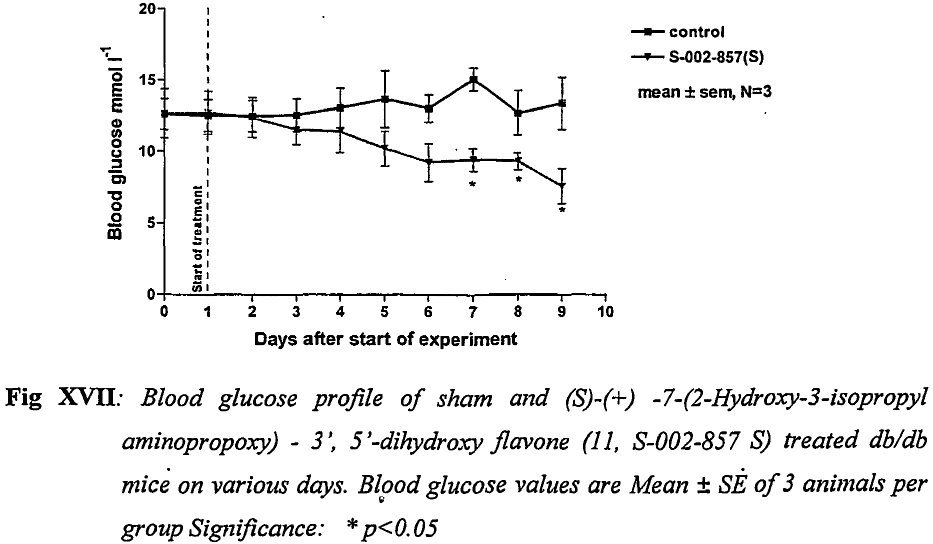

**Table 11: Blood glucose profile of sham and (S)-(+) -7-(2-Hydroxy-3-isopropyl aminopropoxy)-3', 5'-dihydroxy flavone (11, S-002-857 S) treated db/db mice post oral glucose load.**

| . | **Blood glucose (mM) min post glucose load** | | | | | |
|---|---|---|---|---|---|---|
| | **-30** | **0** | **30** | **60** | **90** | **120** |
| **Sham treated** | 12.9 ±2.70 | 17.6 ±5.59 | 33.2 ±0.19 | 27.2 ±1.54 | 22.3 ±0.55 | 18.7 ±1.64 |
| **S-002-857 (S) treated (25 mg/kg)** | 8.76 ±1.90 | 13.8 ±2.18 | 25.3 ±0.58 | 19.8 ±1.20 | 15.2 ±0.48 | 11.6 ±0.58 |
| **% change compared to control** | **-32.2** | **-21.1** | **-23.7** | **-26.9** | **-31.5** | **-37.6** |

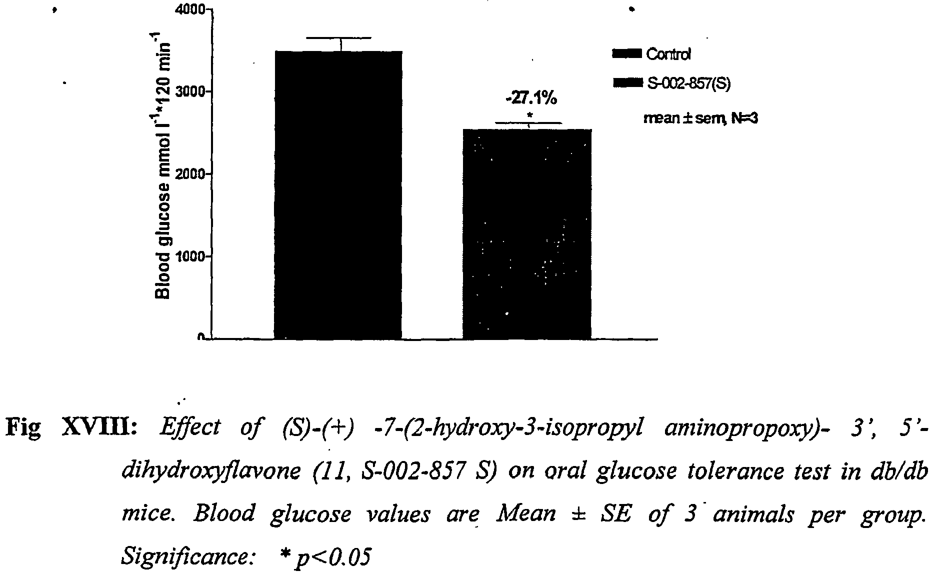

**Table 12: Serum lipid profile in sham and (S)-(+) -7-(2-Hydroxy-3-isopropyl aminopropoxy)-3',5'-dihydroxyflavone (11, S-002-857 S) treated db/db mice.**

| | **Serum lipid profile (mg/dl)** | | |
|---|---|---|---|
| | **Triglycerides** | **Cholesterol** | **HDL-Chol** |
| **Sham treated** | 133±7.00 | 171±4.73 | 56.0±1.00 |
| **S-002-857 (S) (25 mg/kg)** | 124±3.51 | 152±7.00 | 65.0±6.03 |
| **% change compared to control** | **-6.54** | **-11.2** | **+17.3** |

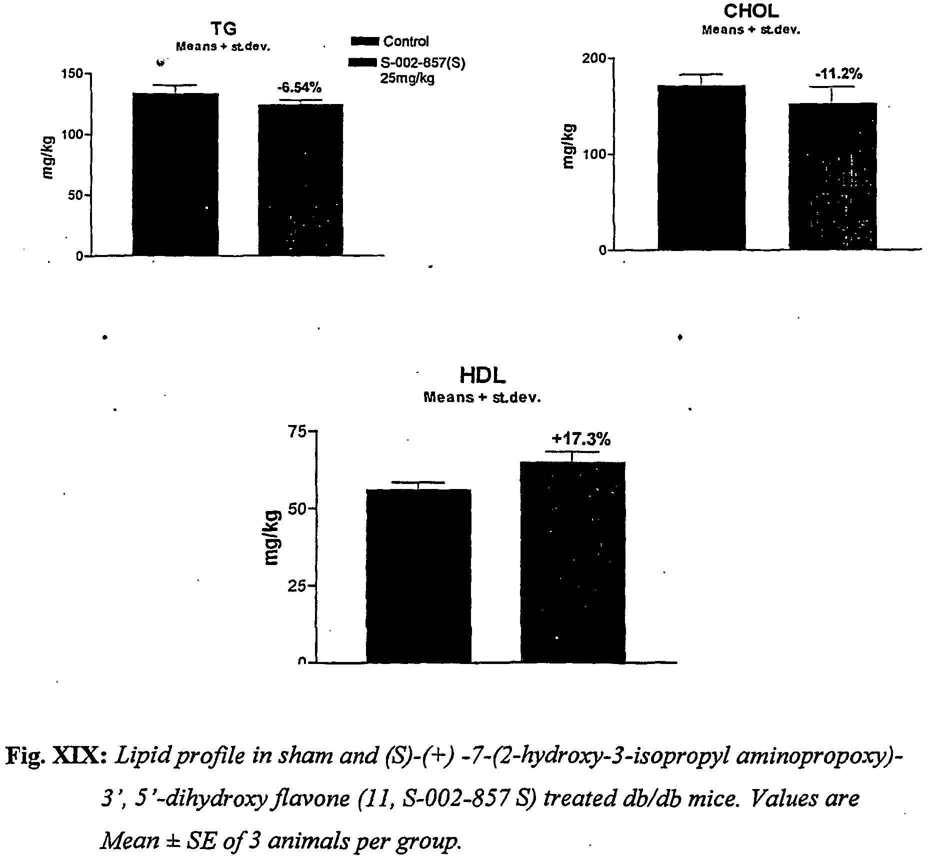

*3', 5'-dihydroxy flavone (11, S-002-857 S) treated db*/*db mice. Values are*
*Mean ± SE of 3 animals per group.*

### (R)- (-) - 7-(2-Hydroxy-3-isopropyl aminopropoxy)- 3',5'-dihydroxy flavone (9, S-002-857 R):

Table 13 presents the blood glucose profile of sham and 25.0 mg/kg S-002-857 R treated db/db mice. A marginal fall in blood glucose profile was observed from day 4th in S-002-857 R treated db/db mice compared to sham treated ones, which more or less remained the same till the end of the study. Figure XX is the graphical presentation of blood glucose profile versus days of treatment of vehicle and S-002-857 R at 25 mg/kg dose level, respectively. There was no significant effect on blood glucose profile was observed on any day post treatment. Table 14 and figure XXI presents the effect of S-002-857 (R) on glucose tolerance of the db/db mice. Though, slight improvement in glucose tolerance was observed in db/db mice treated with 25.0 mg/kg S-002-857 R but it was not found statistically significant.

Table 15 and figure XXII present the lipid profiles of sham and S-002-857 R treated db/db mice. There was found no statistically significant difference between the serum cholesterol, triglycerides and HDL-cholesterol levels of sham and 25.0 mg/kg S-002-857 R treated db/db mice.

**Table 13: Blood glucose profile of (R)-(-)-7-(2-hydroxy-3-isopropyl aminopropoxy)-3',5'-dihydroxyflavone (9, S-002-857 R) treated db/db mice compared to sham treated control.**

| | **Blood glucose (mM) days post treatment** | | | | | | | | | |
|---|---|---|---|---|---|---|---|---|---|---|
| | **1** | **2** | **3** | **4** | **5** | **6** | **7** | **8** | **9** | **10** |
| **Sham treated** | 12.5 ±1.94 | 12.4 ±1.90 | 12.5 ±1.97 | 13.0 ±2.40 | 13.6 ±3.43 | 13.0 ±1.65 | 15.0 ±1.39 | 1.2.7 ±2.69 | 13.3 ±3.18 | 12.9 ±2.70 |
| **S-002-857 R treated (25 mg/kg)** | 12.5 ±2.55 | 12.3 ±2.67 | 12.1 ±2.02 | 11.5 ±1.86 | 11.9 ±2.74 | 11.6 ±2.73 | 11.7 ±1.78 | 11.0 ±0.35 | 11.5 ±1.00 | 11.9 ±5.21 |
| **% change compared to control** | **0.0** | **-1.25** | **-3.28** | **-11.5** | **-13.1** | **-10.7** | **-22.0** | **-13.4** | **-14.0** | **-8.30** |
| **Statistical significance** | **ns** | **ns** | **ns** | **ns** | **ns** | **ns** | **ns** | **ns** | **ns** | **ns** |

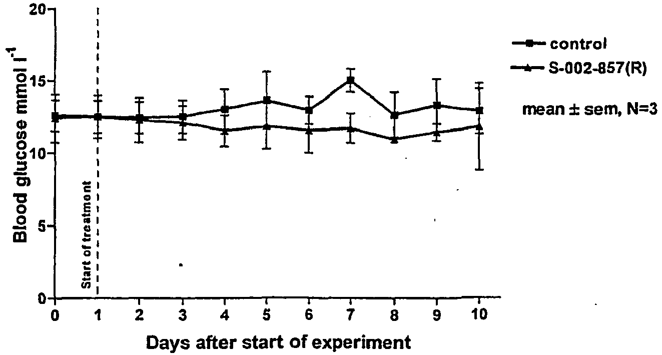

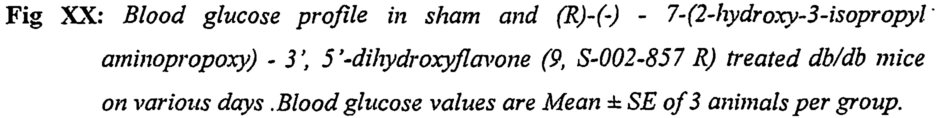

**Table 14: Blood glucose profile of sham and (R)-(-)-7-(2-hydroxy-3-isopropyl aminopropoxy)-3',5'-dihydroxyflavone (9, S-002-857 R) treated db/db mice post oral glucose load.**

| | **Blood glucose profile (mM) min post glucose load** | | | | | |
|---|---|---|---|---|---|---|
| | **-30** | **0** | **30** | **60** | **90** | **120** |
| **Sham treated** | 12.9 ±2.70 | 17.6 ±5.59 | 33.2 ±0.19 | 27.2 ±1.54 | 22.3 ±0.55 | 18.7 ±1.64 |
| **S-002-857 (R) treated (25 mg/kg)** | 11.8 ±5.21 | 16.3 ±3.91 | 27.6 ±1.53 | 24.4 ±2.99 | 20.6 ±2.08 | 16.0 ±1.73 |
| **% change compared to control** | **-8.31** | **-7.05** | **-16.7** | **-10.0** | **-7.39** | **-14.3** |

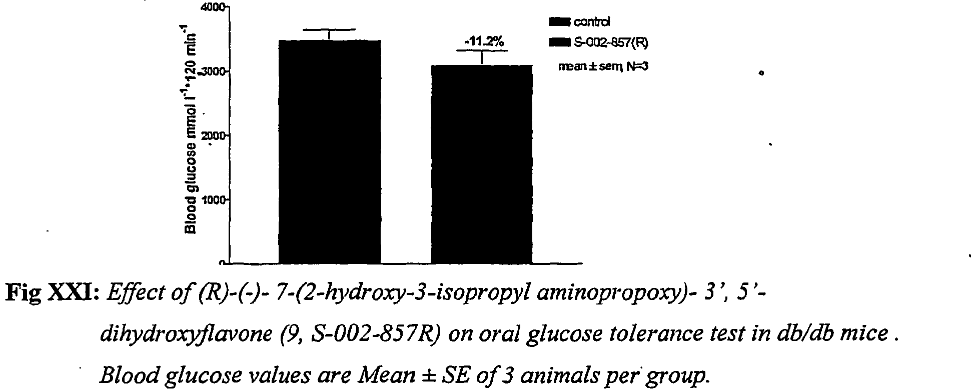

**Table 15: Serum lipid profile in sham and (R)-(-) - 7-(2-Hydroxy-3-isopropyl aminopropoxy)-3',5'-dihydroxy flavone (9, S-002-857 R) treated db/db mice.**

| | **Serum lipid profile (mg/dl)** | | |
|---|---|---|---|
| | **Triglycerides** | **Cholesterol** | **HDL-Chol** |
| **Sham treated** | 133±7.00 | 171±4.73 | 56.0±1.00 |
| **S-002-857 (R) treated (25 mg/kg)** | 130±3.06 | 160±6.66 | 59.0±2.65 |
| **% change compared to control** | **-1.72** | **-6.18** | **+5.35** |

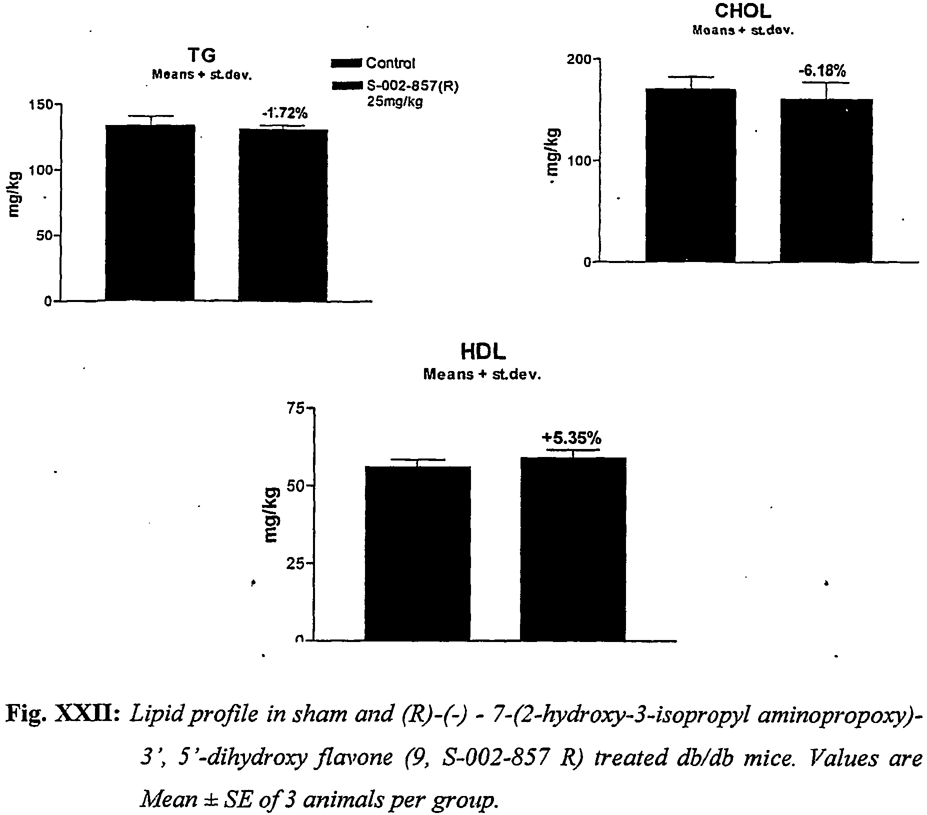

Table 16 and 17 presents the comparative antihyperglycemic and antihyperlipidemic profiles of racemic mixture and the two isomers of S-002-853 in db/db mice. It is evident from the data that S-isomer has marked antihyperglycemic and antihyperlipidemic effect compared to racemic mixture at 25.0 mg/kg dose. R-isomer has insignificant antihyperglycemic or antihyperlipidemic effects

**Table 16. Comparative antihyperlipidemic activity of S-002 853 (S), S-002 853 (R) with S-002-853 (racemic mixture)**

| **Test dose (mg/kg)** | **S-002-853 (Racemic)** | | | **S-002-853 (S)** | | | **S-002-853 (R)** | | |
|---|---|---|---|---|---|---|---|---|---|
| | **TG** | **T-Chol** | **HDL-C** | **TG** | **T-Chol** | **HDL-C** | **TG** | **T-Chol** | **HDL-C** |
| 10 | 13.9* | 824 | +3.03^{ns} | 17.9* | 163* | +17.4^{ns} | - | - | - |
| 25 | -10.1* | -4.47 | +16.2^{ns} | 14.5** | 27.8* | +33.2* | -9.72^{ns} | -3.72^{ns} | +8.06^{ns} |

| | | | | | | | | | |
|---|---|---|---|---|---|---|---|---|---|
| **Significance *p<0.05** p<0.01 ns: not significant** | | | | | | | | | |

**Table 17. Comparative antihyperglycemic activity of S-002 853 (S), S-002 853 (R) with S-002-853 (raceme mixture)**

| **Test dose (mg/kg)** | **S-002-853 (Racemic)** | | **S-002-853 (S)** | | **S-002-853 (R)** | |
|---|---|---|---|---|---|---|
| | **Average Fall** | **Day 10 (OGTT)** | **Average Fall** | **Day 10 (GTT)** | **Average Fall** | **Day 10 (OGTT)** |
| 10 | 44.6* (day 6) | 15.4* | 42.2* (day 8) | 27.6* | ND | ND |
| 25 | 57.7** (day 7) | 35.8* | 61.1** (day 6) | 45.3** | 38.3^{ns} | +4.24^{ns} |

| | | | | | | |
|---|---|---|---|---|---|---|
| **ND: Not done, Significance *p<0.05 **p<0.01 ns: not significant** | | | | | | |

## Claims

1. An optically active compound selected from the group consisting of:
(a) S-(+)-7-(3-tert-Butylamino-2-hydroxy-propoxy)-3',5'-dibenzyloxy-flavone (7);
(b) S-(+)-7-(3-iso-Propylamino-2-hydroxy-propoxy)-3',5'-dibenzyloxy-flavone (10); or
(c) S-(+)-7-(3-iso-Propylamino-2-hydroxy-propoxy)-3',5'-dihydroxy-flavone (11).

2. A compound according to claim 1, wherein the compound is;

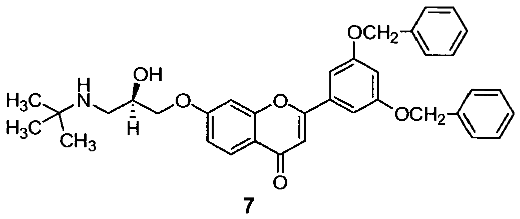

3. A compound according to claim 1, wherein the compound is;

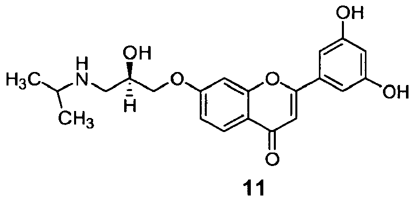

4. A compound according to claim 1, wherein the salt of the compound is selected from group consisting of hydrochloride, succinates, fumarates and tartrates.

5. A compound according to claim 1, wherein the compound exhibits antidiabetic and lipid lowering activity at a dose ranging between 10-250 mg /Kg-body weight.

6. A process for preparation of a compound according to claim 1, comprising the steps of:
(i) reacting substituted hydroxyflavone of general formula **A** wherein R₁, R₂ and R₃ are selected from the group consisting of hydrogen, methyl or iso pentenyl; wherein R₄, R₅ and R₆ are selected from the group consisting of O-alkyl, O-benzyl, O-substituted hydroxy, phenyl or combination thereof, with optically active S *epi*chlorohydrin followed by reaction with a base to obtain the inverted 2,3-epoxy propoxy-flavones of compound of formula **B,** 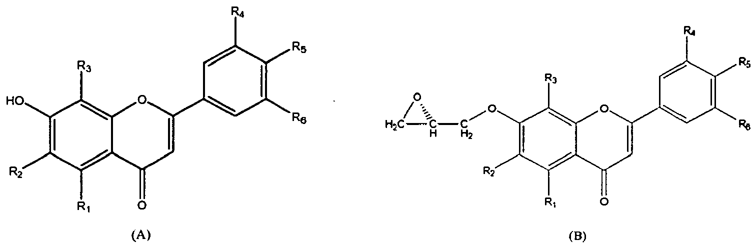
(ii) heating the 2,3-epoxy-propoxy-flavones of the formula B as obtained in step (i) under reflux, with an amine in an alcohol to yield propanolamines of formula C wherein R is selected from a group consisting of t-butyl amine, n-butylamine, isobutylamine, iso-propyl amine, 4-phenyl-piperazine-1-ylamine, 4-(2-methoxyphenyl)-piperazin-1-ylamine, and 3, 4-dimethoxy phenethyl amine, 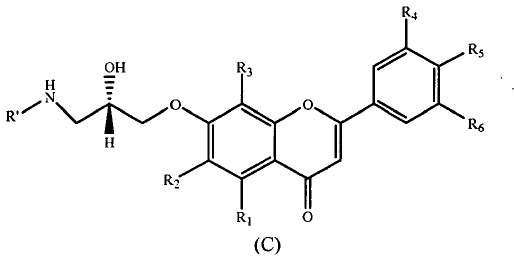
(iii) subjecting the propanolamine obtained in step (ii) for debenzylation using catalytic hydrogenation process to yield corresponding hydroxy flavone derived propanolamines of general formula **C** wherein R₄, R₅, and R₆ are selected from the group of H, OH and O-methoxy. 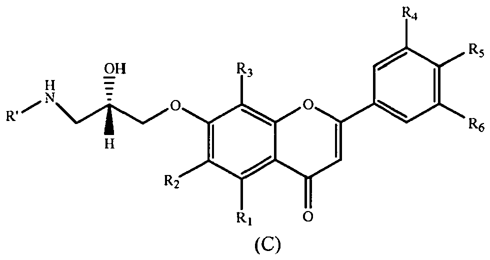
(iv) converting the compound of formula C into salts by known method, preferably wherein the base is selected from the group consisting of sodium hydroxide, potassium hydroxide and benzyltriethylammonium hydroxide.

7. A process according to claim 6, wherein the amine used is selected from the group consisting of alkylamines from a group of cyclic and acyclic alkyl part.

8. A process according to claim 6, wherein the alcohol used is selected from the group consisting of ethanol and propanol.

9. A process according to claim 6, wherein the debenzylation is carried out sing Pd-C as catalyst under hydrogen pressure ranging between 40-100 psi (276-690 kPa).

10. A process according to claim 6, wherein the salt of general formula is selected from the group consisting of hydrochloride, fumarate and tartrates.

11. A compound according to any one of claims 1 to 5 for use in a method of treating type II diabetes and dislipidemia in mammals, said method comprising the step of administering a pharmaceutically effective amount of the compound, optionally with the pharmaceutically acceptable exciepients.

12. The compound according to claim 11, wherein the pharmaceutically effective amount of the compound is in the range of 10-100 mg. /kg-body weight.

13. The method according to claim 11, wherein ED₅₀ of the compound is in the range of 17.84 to 32.93 mg/kg.

## Patentansprüche

1. Optisch aktive Verbindung, die aus der Gruppe ausgewählt ist, die aus Folgendem besteht:
(a) S-(+)-7-(3-tert.-Butylamino-2-hydroxypropoxy)-3',5'-dibenzyloxyflavon (7);
(b) S-(+)- 7-(3-Isopropylamino-2-hydroxypropoxy)-3',5'-dibenzyloxyflavon (10) oder
(c) S-(+)-7-(3-Isopropylamino-2-hydroxypropoxy)-3',5'-dihydroxyflavon (11).

2. Verbindung nach Anspruch 1, wobei die Verbindung folgende Verbindung ist:

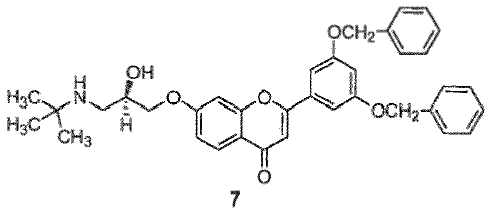

3. Verbindung nach Anspruch 1, wobei die Verbindung folgende Verbindung ist:

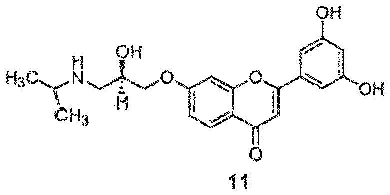

4. Verbindung nach Anspruch 1, wobei das Salz der Verbindung aus der Gruppe bestehend aus Hydrochlorid, Succinaten, Fumaraten und Tartraten ausgewählt ist.

5. Verbindung nach Anspruch 1, wobei die Verbindung bei einer Dosis, die von 10 - 250 mg/kg Körpergewicht reicht, eine antidiabetische und lipidsenkende Wirkung zeigt.

6. Verfahren zur Herstellung einer Verbindung nach Anspruch 1, das die folgenden Schritte umfasst:
(i) Umsetzen von substituiertem Hydroxyflavon der allgemeinen Formel **A,** wobei R₁, R₂ und R₃ aus der Gruppe bestehend aus Wasserstoff, Methyl oder Isopentenyl ausgewählt sind; wobei R₄, R₅ und R₆ aus der Gruppe bestehend aus O-Alkyl, O-Benzyl, O-substituiertem Hydroxy, Phenyl oder einer Kombination davon ausgewählt sind, mit optisch aktivem **S-**Epichlorhydrin, gefolgt von einer Reaktion mit einer Base, um die invertierten 2,3-Epoxypropoxyflavone einer Verbindung der Formel **B** zu erhalten: 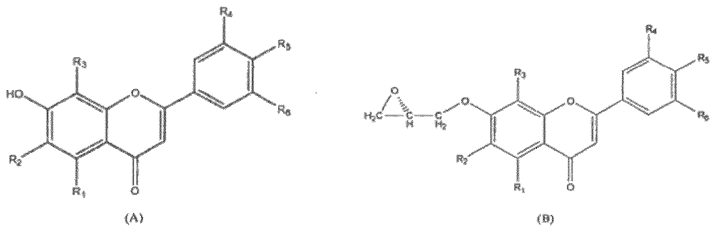
(ii) Erhitzen der wie in Schritt (i) erhaltenen 2,3-Epoxypropoxyflavone der Formel **B unter** Rückfluss mit einem Amin in einem Alkohol, um Propanolamine der Formel **C** zu liefern, wobei R' aus einer Gruppe bestehend aus t-Butylamin, n-Butylamin, Isobutylamin, Isopropylamin, 4-Phenylpiperazin-1-ylamin, 4-(2-Methoxyphenyl)piperazin-1-ylamin und 3,4-Dimethoxyphenethylamin ausgewählt ist: 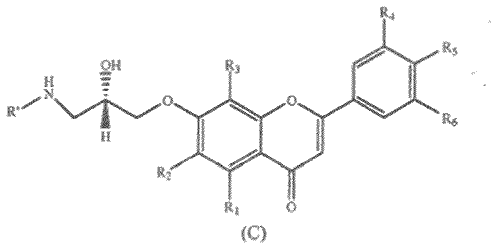
(iii) Unterziehen des in Schritt (ii) erhaltenen Propanolamins einer Entbenzylierung unter Verwendung eines katalytischen Hydrierverfahrens, um entsprechende von Hydroxyflavon abgeleitete Propanolamine der allgemeinen Formel C zu liefern, wobei R₄, R₅ und R₆ aus der Gruppe aus H, OH und O-Methoxy ausgewählt sind: 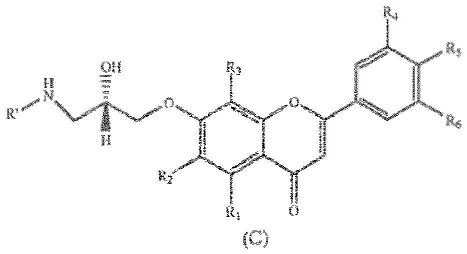
(iv) Umwandeln der Verbindung der Formel **C** in Salze mittels einer bekannten Methode, vorzugsweise wobei die Base aus der Gruppe bestehend aus Natriumhydroxid, Kaliumhydroxid und Benzyltriethylammoniumhydroxid ausgewählt ist.

7. Verfahren nach Anspruch 6, wobei das verwendete Amin aus der Gruppe bestehend aus Alkylaminen von einer Gruppe mit cyclischem und acyclischem Alkylteil ausgewählt ist.

8. Verfahren nach Anspruch 6, wobei der verwendete Alkohol aus der Gruppe bestehend aus Ethanol und Propanol ausgewählt ist.

9. Verfahren nach Anspruch 6, wobei die Entbenzylierung unter Verwendung von Pd-C als Katalysator unter einem Wasserstoffdruck, der von 40 - 100 psi (276 - 690 kPa) reicht, durchgeführt wird.

10. Verfahren nach Anspruch 6, wobei das Salz der allgemeinen Formel aus der Gruppe bestehend aus Hydrochlorid, Fumarat und Tartraten ausgewählt ist.

11. Verbindung nach einem der Ansprüche 1 bis 5 zur Verwendung in einem Verfahren zur Behandlung von Typ-II-Diabetes und Dyslipidämie in Säugetieren, wobei das Verfahren den Schritt des Verabreichens einer pharmazeutisch wirksamen Menge der Verbindung, gegebenenfalls mit den pharmazeutisch unbedenklichen Hilfsstoffen umfasst.

12. Verbindung nach Anspruch 11, wobei die pharmazeutisch wirksame Menge der Verbindung in dem Bereich von 10 - 100 mg/kg Körpergewicht liegt.

13. Verfahren nach Anspruch 11, wobei der ED₅₀-Wert der Verbindung in dem Bereich von 17,84 bis 32,93 mg/kg liegt.

## Revendications

1. Composé optiquement actif choisi dans le groupe comprenant :
la S-(+)-7-(3-tert-biitylamino-2-hydroxy-propoxy)-3',5'-dibenzyloxy-flavone (7) ;
la S-(+)- 7-(3-iso-propylamino-hydroxy-propoxy)-3',5'-dibenzyloxy-flavone (10) ; ou
la S-(+)-7-(3-iso-propylamino-2-hydroxy-propoxy)-3',5'-dihydroxy-flavone (11).

2. Composé selon la revendication 1, le composé étant :

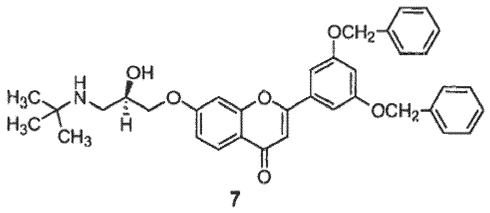

3. Composé selon la revendication 1, le composé étant :

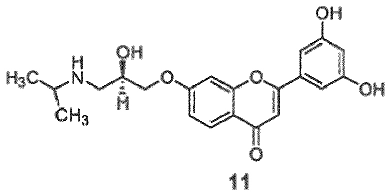

4. Composé selon la revendication 1, dont le sel est choisi dans le groupe comprenant du chlorhydrate, des succinates, des fumarates et des tartrates.

5. Composé selon la revendication 1, le composé présentant une activité antidiabétique et hypolipidémiante à une dose comprise entre 10 et 250 mg/kg de poids corporel.

6. Procédé de préparation d'un composé selon la revendication 1, comprenant les étapes consistant à :
(i) faire réagir l'hydroxyflavone substituée de formule générale **A,** dans lequel R₁, R₂ et R₃ sont choisis dans le groupe comprenant l'hydrogène, le méthyle ou l'isopentényle, et dans lequel R₄, R₅ et R₆ sont choisis dans le groupe comprenant un radical O-alkyle, O-benzyle, O-(hydroxy substitué), phényle ou une de leurs combinaisons, avec de la **S** épichlorohydrine, puis avec une base afin d'obtenir des 2,3-époxy propoxy-flavones inversées du composé de formule **B ;** 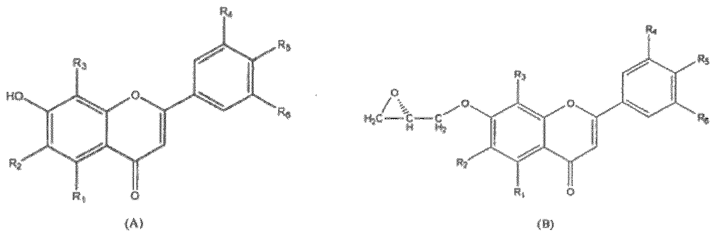
(ii) chauffer les 2,3-époxy-propoxy-flavones de formule **B** obtenues à l'étape (i) par reflux, avec une amine dans un alcool afin d'obtenir des propanolamines de formule **C,** dans lequel R est choisi dans le groupe comprenant la t-butylamine, la n-butylamine, l'isobutylamine, l'isopropylamine, la 4-phénylpipérazine-1-ylamine, la 4-(2-méthoxyphényle)-pipérazine-1-ylamine et la 3,4-diméthoxyphénéthylamine ; 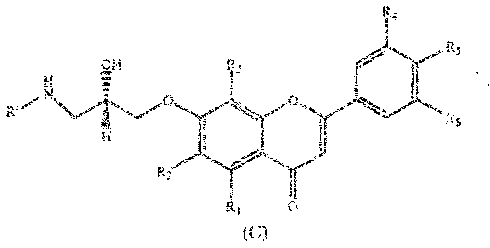
(iii) soumettre la propanolamine obtenue à l'étape (ii) à une débenzylation par un procédé d'hydrogénation catalytique afin d'obtenir les propanolamines correspondantes dérivées de l'hydroxyflavone, de formule générale C, dans lequel R₄, R₅ et R₆ sont choisis dans le groupe comprenant H, OH et un radical O-méthoxy ; 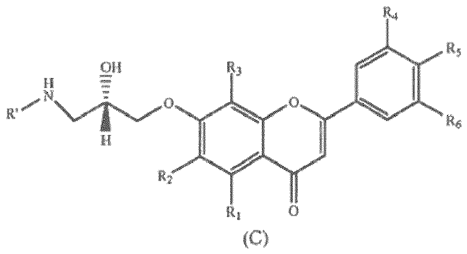
(iv) convertir le composé de formule **C** en sels au moyen de procédés connus, dans lequel la base est de préférence choisie dans le groupe comprenant l'hydroxyde de sodium, l'hydroxyde de potassium et l'hydroxyde de benzyltriéthylammonium.

7. Procédé selon la revendication 6, dans lequel l'amine utilisée est choisie dans le groupe comprenant des alkylamines choisies dans un groupe d'une partie alkyle cyclique et acyclique.

8. Procédé selon la revendication 6, dans lequel l'alcool utilisé est choisi dans le groupe comprenant l'éthanol et le propanol.

9. Procédé selon la revendication 6, dans lequel la débenzylation est réalisée au moyen de Pd-C utilisé comme catalyseur sous une pression d'hydrogène comprise entre 40 et 100 psi (276 et 690 kPa).

10. Procédé selon la revendication 6, dans lequel le sel de formule générale est choisi dans le groupe comprenant du chlorydrate, des fumarates et des tartrates.

11. Composé selon l'une quelconque des revendications 1 à 5 destiné à être utilisé dans un procédé de traitement des diabètes de type II et de la dyslipidémie chez les mammifères, ledit procédé comprenant les étapes consistant à administrer une quantité pharmaceutiquement efficace du composé, éventuellement avec des excipients pharmaceutiquement acceptables.

12. Composé selon la revendication 11, dans lequel la quantité pharmaceutiquement acceptable du composé est comprise entre 10 et 100 mg/kg de poids corporel.

13. Procédé selon la revendication 11, dans lequel la dose efficace moyenne (ED₅₀) du composé est comprise entre 17,84 et 32,93 mg/kg.
